# EUROPEAN PATENT APPLICATION

(11) **EP 4 394 785 A1**
(43) Date of publication of application: **03.07.2024**
(21) Application number: 23220335.6
(22) Date of filing: 27.12.2023
(51) Int. Cl.: G16H 15/00, G16H 40/20, G06Q 10/0633

(54) **SYSTEMS AND METHODS FOR EVALUATING A MEDICAL WORKFLOW**

(30) Priority: 27.12.2022 US 202263477371 P
(71) Applicant: Stryker Corporation, Kalamazoo, MI 49002 (US)
(72) Inventor: TIWARY, Vijay Kumar, Kalamazoo, 49002 (US); KUMAR, Avinash, Kalamazoo, 49002 (US); MEHTA, Sudhanshu, Kalamazoo, 49002 (US); BOCCOLERI, Gianna, Kalamazoo, 49002 (US); BHARDWAJ, Gaurav, Kalamazoo, 49002 (US); GUREVICH, Lina, Kalamazoo, 49002 (US)
(74) Representative: V.O.

(57) **Abstract**

Disclosed herein are methods, systems, and programming for evaluating a medical workflow. For example, one or more images depicting a medical environment and a medical workflow performed in the medical environment may be received. Contextual information associated with the medical workflow may be determined, and a stage of the medical workflow may be identified, based on the one or more images. A subsequent stage of the medical workflow may be determined based on the identified stage, and first medical content associated with the contextual information and second medical content associated with the subsequent stage may be presented to one or more medical staff located within the medical environment.

## Description

### CROSS-REFERENCE TO RELATED APPLICATIONS

This application claims the benefit of U.S. Provisional Application No. 63/477,371, filed December 27, 2022, the entire contents of which are hereby incorporated by reference herein.

### FIELD

The present application generally relates to evaluating a medical workflow and, in particular, using machine learning to improve medical environment safety and efficiency.

### BACKGROUND

Existing operating room dashboards present static content. This content may describe certain aspects of a surgical procedure being performed (e.g., sponge count, blood loss, etc.). These aspects, however, change during the surgical procedure. Existing operating room dashboards update content based on human input. This can hinder and/or prevent medical staff from performing tasks that may need to be completed, thereby decreasing operating room safety and efficiency.

Idle and/or inexperienced medical staff may also contribute to operating room inefficiency. Idle/inexperienced medical staff can increase medical procedure durations, which can negatively impact patient care. Inexperienced medical staff may not know what tasks to perform, when to perform those tasks, or how to perform those tasks. As a result, more experienced medical staff may have to provide instructions or perform those tasks themselves.

Therefore, it would be beneficial to have systems, methods, and programming for intelligently selecting and automatically updating content presented during a surgical procedure to improve operating room safety and efficiency.

### SUMMARY

Described are systems, methods, and programming for evaluating a medical workflow to improve medical environment safety and efficiency. The medical workflow describes different stages associated with a medical procedure. For example, the medical workflow may include one or more stages occurring prior to the medical procedure beginning (e.g., preparing an operating room, bringing in a patient, medical staff entering the operating room, etc.), one or more stages occurring while the medical procedure is performed (e.g., preparing a mixture, cleaning a surface of a medical device within the operating room, making an incision, etc.), and/or one or more stages occurring after the medical procedure has ended (e.g., wheeling a patient out of the operating room, cleaning the operating room, etc.).

Some of these stages may include a set of tasks to be performed by one or more medical staff (e.g., surgeons, nurses, technicians, cleaning staff, etc.). Completion of these tasks may indicate that the current stage has ended and/or a subsequent stage may begin. For example, creating a mixture may include tasks such as measuring the materials, combining the materials, curing the materials, or other tasks. Completion of the tasks may indicate that the mixture has been made. However, not all of the medical staff may know how to perform tasks in a given stage, may be preoccupied with other tasks, or may be idle. This can increase surgical procedure duration, which can negatively impact patient outcomes.

One or more machine learning models may be implemented to intelligently provide medical content to the medical staff to help decrease the amount of time needed to perform certain tasks. The machine learning models may be trained to determine contextual information describing the operating room, identify a current stage of the medical workflow, identify a subsequent stage of the medical workflow, compute an efficiency score, and/or update aspects of the medical workflow to improve the efficiency score. As an example, medical content describing the contextual information (e.g., a user interface displaying an amount of blood lost during the surgery, a number of surgical sponges used, etc.) may be presented to some or all of the medical staff. As another example, medical content describing the subsequent medical stage of the medical workflow (e.g., a video, checklist, audio message, etc.) may be presented to some or all of the medical staff.

A medical workflow efficiency score may be computed indicating the efficiency of the medical staff in carrying out the different stages of the medical workflow. The medical workflow efficiency score may be determined based on the contextual information, the identified medical stage, the subsequent medical stage, or other information. For example, the medical workflow efficiency score may indicate an amount (e.g., a percentage) of time that medical staff were idle during the surgical procedure. Depending on the medical workflow efficiency score, one or more aspects of the medical workflow may be adjusted. For example, the number of medical staff allocated for performances of a given surgical procedure may be adjusted based on the medical workflow efficiency score. This can improve the overall medical workflow efficiency, thereby optimizing resource allocation and improving patient outcomes.

According to some examples, a method for evaluating a medical workflow includes: receiving one or more images depicting a medical environment and a medical workflow performed in the medical environment; determining contextual information associated with the medical workflow based on the one or more images; identifying a stage of the medical workflow based on the one or more images; determining a subsequent stage of the medical workflow based on the identified stage; and presenting, to one or more medical staff located within the medical environment, first medical content associated with the contextual information and second medical content associated with the subsequent stage.

In any of the examples, the method can further include: generating a report for the medical workflow based on the contextual information.

In any of the examples, the medical workflow can comprise a first stage occurring prior to a beginning of a medical procedure, a second stage occurring during the medical procedure, and a third stage occurring after the medical procedure has been completed, wherein identifying the stage of the medical workflow comprises: determining whether the one or more images were captured during the first stage, the second stage, or the third stage.

In any of the examples, the method can further include: inputting the one or more images into one or more machine learning models to obtain one or more image representations.

In any of the examples, identifying the stage of the medical workflow can comprise: identifying activities of the one or more medical staff based on the one or more image representations, the stage of the medical workflow being determined based on the identified activities.

In any of the examples, determining the contextual information can comprise: detecting, based on the one or more image representations, one or more objects present within the one or more images; and generating the contextual information based on the one or more detected objects.

In any of the examples, the method can further include: generating a report comprising at least some of the first medical content, the second medical content, or the first medical content and the second medical content; and presenting, via one or more display devices, the report to the one or more medical staff.

In any of the examples, the method can further include: generating an audio message based on subsequent stage information, the audio message describing the subsequent stage of the medical workflow.

In any of the examples, the method can further include: outputting the audio message describing the subsequent stage to the one or more medical staff.

In any of the examples, presenting the second medical content can comprise: selecting one or more display devices with which to present the second medical content, the one or more display devices being located within the medical environment; and sending the second medical content to the one or more selected display devices.

In any of the examples, selecting the one or more display devices can comprise: identifying, based on the one or more images, one or more relevant medical staff located within the medical environment, the one or more relevant medical staff associated with the subsequent stage; and identifying, based on the one or more images, one or more displays located within the medical environment, the one or more displays proximate to the one or more relevant medical staff.

In any of the examples, identifying the one or more relevant medical staff can comprise: detecting, using one or more machine learning models, activities of the one or more relevant medical staff based on the one or more images, the one or more relevant medical staff being identified from the one or more medical staff based on the detected activities.

In any of the examples, the method can further include: detecting, using one or more machine learning models, activities of the one or more medical staff during the stage of the medical workflow based on the one or more images; and generating a report for the medical workflow based on the detected activities.

In any of the examples, generating the report can comprise: generating an efficiency score indicating an efficiency of the medical workflow, the report comprising the generated efficiency score.

In any of the examples, generating the efficiency score can comprise: determining a number of medical staff associated with the stage of the medical workflow based on the detected activities of the one or more medical staff; and comparing the number of medical staff to a predefined number of medical staff to be used for the stage of the medical workflow, the efficiency score being based at least in part on a result of the comparison.

In any of the examples, the method can further include: detecting, using one or more machine learning models, activities of the one or more medical staff during the stage of the medical workflow based on the one or more images; updating at least one of the first medical content or the second medical content based on the activities; and presenting, using one or more display devices, at least one of the updated first medical content or the updated second medical content to the one or more medical staff.

In any of the examples, the method can further include: determining, based on the contextual information, that the medical workflow has progressed from a first stage to a second stage; and updating the first medical content based on the medical workflow progressing from the first stage to the second stage.

In any of the examples, presenting the first medical content and the second medical content can comprise: identifying at least one of (i) one or more checklists associated with the medical workflow or (ii) one or more videos associated with the medical workflow; and generating at least one of the first medical content or the second medical content based on the at least one of (i) the one or more checklists or (ii) the one or more videos.

In any of the examples, the method can further include: identifying, based on the one or more images, a current task of a plurality of tasks associated with the identified stage; determining, based on the one or more images, that the current task has been completed; and modifying a checklist associated with the identified stage to indicate that the current task has been completed.

In any of the examples, the method can further include: identifying, from a plurality of tasks associated with the identified stage, a current task and a subsequent task; determining, based on the one or more images, that the subsequent task has been started by the one or more medical staff prior to completion of the current task; and sending a notification to the one or more medical staff indicating that the current task has not been completed.

In any of the examples, sending the notification to the one or more medical staff can comprise: generating and outputting at least one of an audible alert, a visual alert, or a haptic alert to the one or more medical staff.

In any of the examples, the method can further include: detecting, using one or more machine learning models, activities of the one or more medical staff during the stage of the medical workflow based on the one or more images; and generating a report indicating that a quantity of the one or more medical staff is to be adjusted.

In any of the examples, the quantity of the one or more medical staff being adjusted can comprise: increasing the quantity of medical staff for the identified stage; or decreasing the quantity of medical staff for the identified stage.

According to some examples, a system includes: one or more processors programmed to perform the method of any of the examples.

According to some examples, a non-transitory computer-readable medium stores computer program instructions that, when executed, effectuate operations including the method of any of the examples.

According to some examples, computer program product comprises software code portions comprising instructions that, when executed, effectuate operations including the method of any of the examples.

It will be appreciated that any of the variations, aspects, features, and options described in view of the methods apply equally to the systems and computer program products, and vice versa. It will also be clear that any one or more of the above variations, aspects, features, and options can be combined.

### BRIEF DESCRIPTION OF THE FIGURES

The invention will now be described, by way of example only, with reference to the accompanying drawings, in which:
FIG. 1A illustrates an example medical environment, according to some aspects.
FIG. 1B illustrates an example system for evaluating a medical workflow, according to some aspects.
FIG. 2 illustrates an example medical workflow and medical content associated with the medical workflow, according to some aspects.
FIG. 3 illustrates an example machine learning pipeline for determining contextual information and medical stage information, according to some aspects.
FIGS. 4A-4B illustrate example machine learning models for determining contextual information and medical stage information, respectively, according to some aspects.
FIGS. 5A-5B illustrate example medical content presented using one or more client devices, according to some aspects.
FIG. 5C illustrates example contextual information, according to some aspects.
FIG. 6 illustrates an example system for selecting medical content associated with a subsequent medical stage, according to some aspects.
FIGS. 7A-7B illustrate example medical stage information, according to some aspects.
FIG. 8 illustrates an example system of display devices and content to be presented using those display devices, according to some aspects.
FIG. 9 illustrates a flowchart of an example process for evaluating a medical workflow, according to some aspects.
FIG. 10 illustrates a flowchart of an example process for adjusting aspects of a medical workflow based on a medical workflow efficiency score, according to some aspects.
FIG. 11 illustrates an example computing system used for performing any of the techniques described herein, according to some aspects.

### DETAILED DESCRIPTION

Reference will now be made in detail to implementations and various aspects and variations of systems and methods described herein. Although several example variations of the systems and methods are described herein, other variations of the systems and methods may include aspects of the systems and methods described herein combined in any suitable manner having combinations of all or some of the aspects described.

Described are systems, methods, and programming for evaluating a medical workflow. This evaluation may be used to improve operating room efficiency. Operating room efficiency can depend on a variety of factors. For example, medical staff who are inexperienced with certain tasks to be performed may decrease operating room efficiency. As another example, medical staff that remain idle when tasks could be performed can also decrease operating room efficiency. As yet another example, operating room dashboards that present static content can decrease operating room efficiency. Greater operating room efficiency can help improve patient care during the medical procedure and may also improve medical procedure outcomes.

To improve operating room efficiency, one or more machine learning models may be used to intelligently identify a current stage of a medical workflow and retrieve content associated with the current stage and/or a subsequent stage. Additionally, one or more machine learning models may be used to extract contextual information about the medical procedure from operating room images. This contextual information can be used to dynamically update the content presented via operating room dashboards.

The presented content may include checklists of tasks to be performed. The tasks may help facilitate compliance with the medical workflow's requirements. The presented content may also include images, text, and/or videos describing how to perform one or more of the tasks.

The presented content can be utilized by idle roles - medical staff identified as being idle - to assist other medical staff and improve operating room efficiency. For example, the presented content can inform idle medical staff what tasks are to be performed by non-idle medical staff. This may enable those individuals previously identified as idle to provide aid to the other medical staff. Furthermore, the presented content can inform medical staff what should be transpiring within the medical environment, thereby providing improved oversight, and moreover, improving patient outcomes.

Images and/or video of the operating room may be obtained and analyzed via the one or more machine learning models. The one or more machine learning models may be trained to identify objects within the operating room as well as actions, if any, occurring within the operating room. For example, the machine learning models may detect medical sponges and/or blood loss and compute a quantity of medical sponges used and/or blood lost during a surgical procedure. As another example, the machine learning models may detect medical staff within the operating room and surgical activities, if any, being performed by the medical staff.

The outputs from the machine learning models may be used to dynamically update content presented to the medical staff. For example, contextual information describing changes in the quantity of medical sponges used and/or blood lost during the surgery can be determined using the machine learning models. Content presented to the medical staff may be dynamically updated based on changes in contextual information. For example, the content may be updated based on activities of users within the medical environment. The updated content can improve operating room efficiency as compared to conventional techniques. For example, instead of (or in addition to) manually tracking and inputting contextual information to update the presented content, the machine learning models may be configured to detect changes in contextual information and update the presented content automatically. Medical staff may also monitor these changes (e.g., medical sponge count, blood loss) and can compare their findings with the determinations from the machine learning models. Discrepancies between the machine learning models' determinations and the medical staff's findings may be further investigated to identify the cause of the discrepancies and mitigate the discrepancies (e.g., by re-training one or more of the machine learning models).

A report for the medical workflow may be generated describing the presented content, the contextual information, the identified stage, the subsequent stage, and/or other information. For example, a report may be generated that describes discrepancies found between the machine learning models' determinations (e.g., the contextual information) and the medical staff's finding.

As another example, the activities performed by the medical staff may be used to determine stages of the medical workflow. These stages may each have associated content that is to be presented to some or all of the medical staff. By identifying the current stage, content associated with the current stage as well as the known subsequent stage may be retrieved in advance and presented to the medical staff. This can improve operating efficiency by providing medical staff with the necessary information to perform tasks associated with each stage, thereby minimizing idleness and optimizing medical staff resource allocation.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes, "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or display devices.

The present disclosure in some examples also relates to a device for performing the operations herein. This device may be specially constructed for the required purposes, or it may comprise a general-purpose computer selectively activated or reconfigured by a computer program stored in the computer. Such a computer program may be stored in a non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability. Suitable processors include central processing units (CPUs), graphical processing units (GPUs), field-programmable gate arrays (FPGAs), and ASICs.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

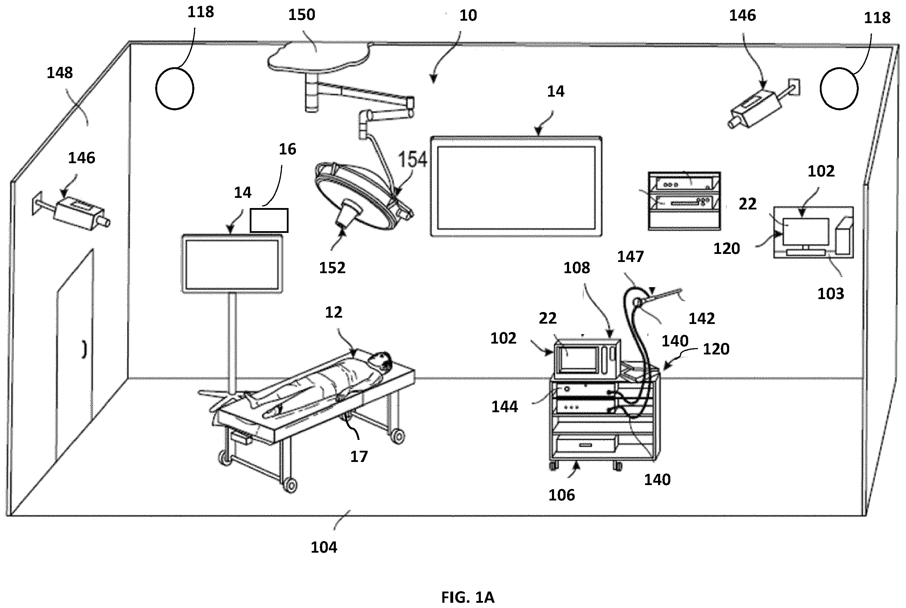

FIG. 1A illustrates an example medical environment 10, according to some aspects. Medical environment 10 may represent a surgical suite, operating room, or other care environment where a medical procedure may be performed. Furthermore, activities may be performed within medical environment 10 that occur prior to or after a medical procedure has been performed. For example, activities that may occur prior to the medical procedure beginning may include, but are not limited to, setting up medical equipment, preparing mixtures, cleaning surfaces/objects, wheeling a patient into medical environment 10, placing a patient on a surgical table 17, and/or dispensing anesthetics. As another example, activities that may occur after the medical procedure has ended may include moving a patient to a stretcher or wheelchair, wheeling the patient out of medical environment 10, cleaning surfaces/objects, or moving medical devices to different locations.

Medical environment 10 may include devices used to prepare for and/or perform a medical procedure to a patient 12. These devices may also be used after the medical procedure. Such devices may include one or more sensors, one or more medical devices, one or more display devices, one or more light sources, one or more computing devices, or other components. For example, at least one medical device 120 may be located within medical environment 10. Medical device 120 may be used to assist medical staff while performing a medical procedure (e.g., surgery). Medical device 120 may also be used to document events and information from the medical procedure. For example, medical device 120 may be used to input or receive patient information (e.g., to/from electronic medical records (EMRs), electronic health records (EHRs), hospital information system (HIS), communicated in real-time from another system, etc.). The received patient information may be saved onto medical device 120. Alternatively or additionally, the patient information may be displayed using medical device 120. In some aspects, medical device 120 may be used to record patient information. For example, medical device 120 may be used to store the patient information or images in an EMR, EHR, HIS, or other databases.

Medical device 120 may be capable of obtaining, measuring, detecting, and/or saving information related to the patient 12. Medical device 120 may or may not be coupled to a network that includes records of patient 12, for example, an EMR, EHR, or HIS. Medical device 120 may include or be integrated with a computing system 102 (e.g., a desktop computer, a laptop computer, a tablet device, etc.) having an application server. For example, medical device 120 may include processors or other hardware components that enable data to be captured, stored, saved, and/or transmitted to other devices. Computing system 102 can have a motherboard that includes one or more processors or other similar control devices as well as one or more memory devices. The processors may control the overall operation of computing system 102 and can include hardwired circuitry, programmable circuitry that executes software, or a combination thereof. The processors may, for example, execute software stored in the memory device. The processors may include, for example, one or more general- or special-purpose programmable microprocessors and/or microcontrollers, graphics processing unit (GPU), tensor processing unit (TPU), application specific integrated circuits (ASICs), programmable logic devices (PLDs), programmable gate arrays (PGAs), or the like. The memory devices may include any combination of one or more random access memories (RAMs), read-only memories (ROMs) (which may be programmable), flash memory, and/or other similar storage devices. Patient information may be inputted into computing system 102 (e.g., making an operative note during the medical or surgical procedure on patient 12 in medical environment 10) and/or computing system 102 can transmit the patient information to another medical device 120 (via either a wired connection or wirelessly).

Computing system 102 can be positioned in medical environment 10 on a table (stationary or portable), a floor 104, a portable cart 106, an equipment boom, and/or a shelving 103. FIG. 1A illustrates two forms of computing system 102: a first computing system in the form of a desktop computer on shelving 103 and a second computing system incorporated into an imaging system 108 on portable cart 106. Further, examples of the disclosure may include any number of computer systems.

In some aspects, medical environment 10 may be an integrated suite used for minimally invasive surgery (MIS) or fully invasive procedures. Video components, audio components, and associated routing may be located throughout medical environment 10. For example, monitor 14 may present video and speakers 118 may output audio. The components may be located on or within the walls, ceilings, or floors of medical environment 10. For example, room cameras 146 may be mounted to walls 148 or a ceiling 150. Wires, cables, and hoses can be routed through suspensions, equipment booms, and/or interstitial space. The wires, cables, and/or hoses in medical environment 10 may be capable of connecting to mobile equipment, such as portable cart 106, C arms, microscopes, etc. to communicate routing audio, video, and data information.

Imaging system 108 may be configured to capture images and/or video, and may route audio, video, and other data (e.g., device control data) throughout medical environment 10. Imaging system 108 and/or associated router(s) may route the information between devices within or proximate to medical environment 10. In some aspects, imaging system 108 and/or associated router(s) (not shown) may be located external to medical environment 10 (e.g., in a room outside of an operating room), such as in a closet. As an example, the closet may be located within a predefined distance of medical environment 10 (e.g., within 325 feet, or 100 meters). In some aspects, imaging system 108 and/or the associated router(s) may be located in a cabinet inside or adjacent to medical environment 10.

The captured images and/or videos may be displayed via one or more display devices. For example, images captured by imaging system 108 may be displayed using monitor 14. Imaging system 108, alone or in combination with one or more audio sensors, may also be capable of recording audio, outputting audio, or a combination thereof. In some aspects, patient information can be inputted into imaging system 108 and associated with the images and videos recorded and/or displayed. Imaging system 108 can include internal storage (e.g., a hard drive, a solid-state drive, etc.) for storing the captured images and videos. Imaging system 108 can also display any captured or saved images (e.g., from the internal hard drive). For example, imaging system 108 may cause monitor 14 to display a saved image. As another example, imaging system 108 may display a saved video using a touchscreen monitor 22. Touchscreen monitor 22 and/or monitor 14 may be coupled to imaging system 108 via either a wired connection or wirelessly. It is contemplated that imaging system 108 could obtain or create images of patient 12 during a medical or surgical procedure from a variety of sources (e.g., from video cameras, video cassette recorders, X-ray scanners (which convert X-ray films to digital files), digital X-ray acquisition apparatus, fluoroscopes, computed tomography (CT) scanners, magnetic resonance imaging (MRI) scanners, ultrasound scanners, charge-coupled (CCD) devices, and other types of scanners (handheld or otherwise)). If coupled to a network, imaging system 108 can also communicate with a picture archiving and communication system (PACS), as is well known to those skilled in the art, to save images and videos in the PACS and to retrieve images and videos from the PACS. Imaging system 108 can couple to and/or integrate with, e.g., an electronic medical records database (e.g., EMR) and/or a media asset management database.

Touchscreen monitor 22 and/or monitor 14 may display images and videos captured live by imaging system 108. Imaging system 108 may include at least one image sensor, for example, disposed within video camera 140. Video camera 140 may be configured to capture an image or a sequence of images (e.g., video frames) of patient 12. Video camera 140 can be a hand-held device, such as an open-field camera or an endoscopic camera. For example, imaging system 108 may be coupled to an endoscope 142, which may include or be coupled to video camera 140. Imaging system 108 may communicate with a camera control unit 144 via a fiber optic cable 147, which may communicate with imaging system 108 (e.g., via a wired or wireless connection).

Room cameras 146 may also be configured to capture an image or a sequence of images (e.g., video frames) of medical environment 10. The captured image(s) may be displayed using touchscreen monitor 22 and/or monitor 14. In addition to room cameras 146, a camera 152 may be disposed on a surgical light 154 within medical environment 10. Camera 152 may be configured to capture an image or a sequence of images of medical environment 10 and/or patient 12. Images captured by video camera 140, room cameras 146, and/or camera 152 may be routed to imaging system 108, which may then be displayed using touchscreen monitor 22, monitor 14, another display device, or a combination thereof. Additionally, the images captured by video camera 140, room cameras 146, and/or camera 152 may be provided to a database for storage (e.g., an EMR).

Room cameras 146, camera 152, and/or video camera 140 (or another camera of imaging system 108) may include at least one solid state image sensor. For example, the image sensor of room cameras 146, camera 152, and/or video camera 140 may include a charge coupled device (CCD), a complementary metal-oxide semiconductor (CMOS) sensor, a charge-injection device (CID), or another suitable sensor technology. The image sensor of room cameras 146, camera 152, and/or video camera 140 may include a single image sensor. The single image sensor may be a grayscale image sensor or a color image sensor having an RGB color filter array deposited on its pixels. The image sensor of room cameras 146, camera 152, and/or video camera 140 may alternatively include three sensors: one sensor for detecting red light, one sensor for detecting green light, and one sensor for detecting blue light.

The medical procedure in which the images may be captured using room cameras 146, camera 152, and/or video camera 140 may be an exploratory procedure, a diagnostic procedure, a study, a surgical procedure, a non-surgical procedure, an invasive procedure, or a non-invasive procedure. As mentioned above, video camera 140 may be an endoscopic camera (e.g., coupled to endoscope 142). It is to be understood that the term endoscopic (and endoscopy in general) is not intended to be limiting, and rather video camera 140 may be configured to capture medical images from various scope-based procedures including but not limited to arthroscopy, ureteroscopy, laparoscopy, colonoscopy, bronchoscopy, etc.

Speakers 118 may be positioned within medical environment 10 to provide sounds, such as music, audible information, and/or alerts, that can be played within the medical environment during the medical procedure. For example, speaker(s) 118 may be installed on the ceiling150, installed on the wall 148 and/or positioned on a bookshelf, a station, etc.

One or more microphones 16 may sample audio signals within medical environment 10. The sampled audio signals may comprise the sounds played by speakers 118, noises from equipment within medical environment 10, and/or human speech (e.g., voice commands to control one or more medical devices, verbal information conveyed for documentation purposes, etc.). Microphone(s) 16 may be located within a speaker (e.g., a smart speaker) attached to monitor 14, as shown in FIG. 1A, and/or within the housing of touchscreen monitor 22. Microphones 16 may communicate via a wired or wireless connection with imaging system 108 and/or computing system 102. Microphones 16 may provide, record, and/or process the sampled audio signals. For example, as mentioned above, microphones 16 may determine and record the surgeon's speech for documentation purposes (e.g., recording verbal information for educational purposes, making room calls, sending real-time information to pathologists, etc.). Additionally or alternatively, microphones 16 may be capable of processing the sampled audio signals, including recognizing the voice commands received from the surgeon (or another medical professional). The captured audio signal may also be processed using computing system 102.

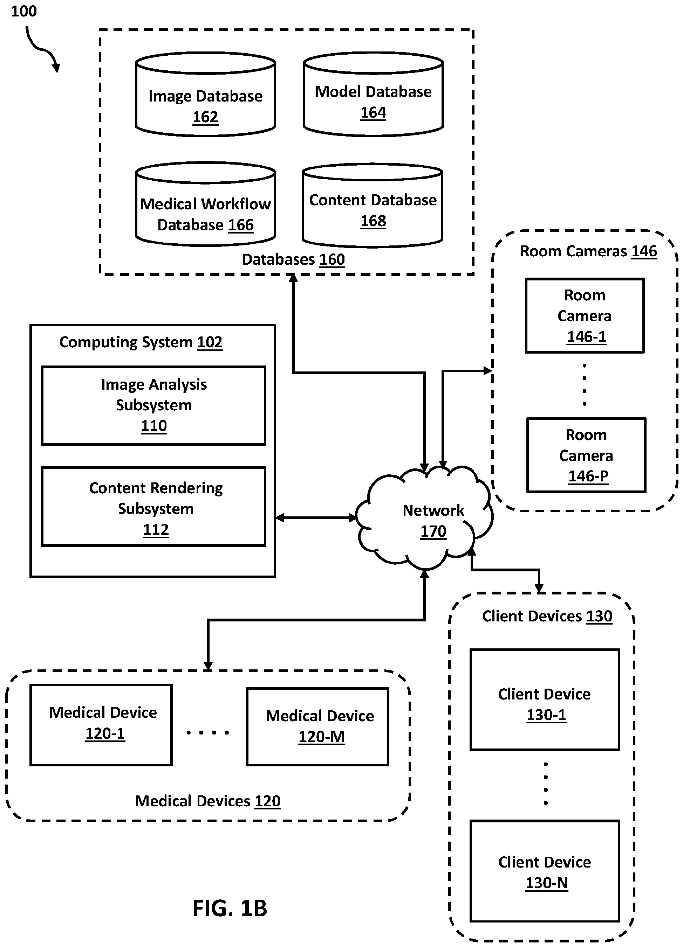

FIG. 1B is a diagram illustrating an example system 100, according to some aspects. System 100 may include computing system 102, medical devices 120 (e.g., medical devices 120-1 to 120-M), client devices 130 (e.g., client devices 130-1 to 130-N), room cameras 146 (e.g., room cameras 146-1 to 146-P), databases 160 (e.g., image database 162, model database 164, medical workflow database 166, content database 168), or other components. Components of system 100 may communicate with one another using network 170 (e.g., the Internet).

Medical devices 120 may include one or more sensors, such as an image sensor, an audio sensor, a motion sensor, or other types of sensors. Image sensors may be configured to capture one or more images, one or more videos, audio data, or other data relating to a medical procedure. As an example, with reference to FIG. 1A, medical device 120 may correspond to endoscope 142. Endoscope 142 may include a video camera 140 having one or more image sensors. Medical device 120 may be used to obtain or create images of patient 12 during a medical procedure from a variety of sources (e.g., from video cameras, video cassette recorders, X-ray scanners (which can convert X-ray films to digital files), digital X-ray acquisition apparatus, fluoroscopes, computed tomography (CT) scanners, magnetic resonance imaging (MRI) scanners, ultrasound scanners, charge-coupled (CCD) devices, and other types of scanners (handheld or otherwise)). For example, medical device 120 may capture images and/or videos depicting an anatomical structure of patient 12, as seen with respect to FIG. 1A. As another example, medical device 120 may be a medical imaging device (e.g., MRI machines, CT machines, X-Ray machines, etc.). As yet another example, medical device 120 may be a biometric data capture device (e.g., a blood pressure device, pulse-ox device, etc.).

Client devices 130-1 to 130-N may be capable of communicating with one or more components of system 100 via a wired and/or wireless connection (e.g., network 170). Client devices 130 may interface with various components of system 100 to cause one or more actions to be performed. For example, client devices 130 may represent one or more devices used to display images and videos to a user (e.g., a surgeon). Examples of client devices 130 may include, but are not limited to, desktop computers, servers, mobile computers, smart devices, wearable devices, cloud computing platforms, display devices, mobile terminals, fixed terminals, or other client devices. Each client device 130-1 to 130-N of client devices 130 may include one or more processors, memory, communications components, display components, audio capture/output devices, imaging components, other components, and/or combinations thereof.

As described above with respect to FIG. 1A, room cameras 146 may include image sensors or devices housing image sensors, which may be capable of capturing images and/or video of medical environment 10. While medical environment 10 illustrated in FIG. 1A includes a plurality of room cameras 146, some medical environments may include a single room camera 146. The images captured by one or more room cameras 146 may be analyzed individually or in combination. For example, a first room camera 146 may capture one or more images of a first portion of medical environment 10, while a second room camera 146 may capture one or more images of a second portion of medical environment 10. These two sets of images may be combined so as to provide a full view of medical environment 10. Room cameras 146 may include processors and memory to analyze and store captured images/videos. For example, room cameras 146 may include a graphical processing unit (GPU) or deep learning processor for executing machine learning models. The captured images/videos may be provided to and stored in image database 162.

In addition to or instead of room cameras 146, camera 152 of FIG. 1A may be used to capture images and/or videos of medical environment 10. For example, camera 152 may capture an image of patient 12 and/or a portion of medical environment 10 adjacent to patient 12. The images and/or videos captured by camera 152 may be analyzed in a manner similar to that described above with respect to room cameras 146. Additionally, the images and/or videos captured by camera 152 may be provided to and stored in image database 162.

Computing system 102 may include one or more subsystems, such as an image analysis subsystem 110, a content rendering subsystem 112, or other subsystems. Subsystems 110, 112 may be implemented using one or more processors (e.g., CPUs, GPUs, TPUs, and the like), memory, and interfaces. Distributed computing architectures and/or cloud-based computing architectures may alternatively or additionally be used to implement at least a portion of the functionalities associated with subsystems 110, 112.

It should be noted that, while one or more operations are described herein as being performed by particular components of computing system 102, those operations may be performed by other components of computing system 102 or other components of system 100. As an example, one or more operations described herein as being performed by components of computing system 102 may alternatively be performed by one or more of medical devices 120 and/or client devices 130.

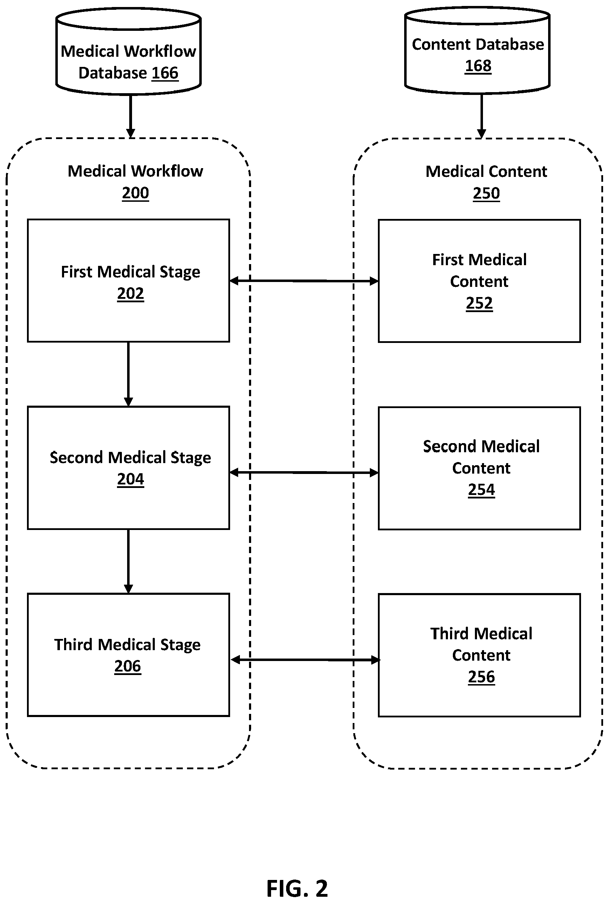

Subsystems 110, 112 may be configured to implement various portions of a medical workflow evaluation process. As an example, with respect to FIG. 2, a medical workflow 200 may include a first medical stage 202, a second medical stage 204, and a third medical stage 206. Different medical workflows 200 may have different quantities of stages, thus the aforementioned workflow is intended to be an example. Furthermore, medical workflow database 166 may store various data structures describing medical workflows 200 associated with medical environment 10. Different medical workflows 200 may be used for different medical environments, different medical procedures performed in those medical environments, and/or different users (e.g., surgeons). Medical workflow 200 may include a medical procedure. Medical workflow 200 may include an amount of time associated with the medical procedure, and/or actions performed prior to the medical procedure, during the medical procedure, and/or after the medical procedure.

First medical stage 202 may correspond to one or more activities performed prior to a medical procedure beginning. For example, first medical stage 202 may include bringing a patient, such as patient 12 of FIG. 1A, into medical environment 10, placing the patient on surgical table 17, cleaning or preparing various medical devices, or other activities. At least a portion of these activities may include one or more tasks that are to be performed. For example, a pre-operative cleaning procedure may include a series of tasks that are to be executed to clean an object (e.g., surgical light 154, surgical table 17, etc.) that may be used during a surgical procedure. Optionally, medical workflow 200 may proceed to a subsequent stage, such as second medical stage 204, based on each of the activities and/or the tasks associated with those activities that have been performed. For example, an activity of first medical stage 202 may include displaying videos, images, or other information that may be used during the medical procedure or may describe the patient, such as patient 12 of FIG. 1A. For example, images, videos, information, and/or checklists may be identified and displayed via monitor 14 to prepare patient 12, medical staff, or other personnel within medical environment 10. The information may include notes related to the medical procedure, such as notes related to the procedure type, specific anatomy, an area of patient 12 to be prepared for the medical procedure and/or a body side (e.g., right side of patient 12, left side of patient 12) to be prepared for the medical procedure.

Returning to FIG. 2, as mentioned above, second medical stage 204 may correspond to one or more activities performed during the medical procedure. These activities may correspond with different steps of the medical procedure. For example, a first step may include making an incision in a patient, whereas a second step may include viewing an anatomical structure. Determining which activity is being performed may indicate the current step of the medical procedure, and the indicated step may be used to determine the current stage of the medical workflow. Some of the activities may include one or more steps to be performed. For example, a mixture may need to be prepared during the surgery. To prepare the mixture, an individual may perform a sequence of steps (e.g., measuring an amount of material, pouring one material into a container, mixing another material into the container, etc.). Computing system 102 of FIG. 1B may be configured to display information in the form of text, diagrams, images, videos, etc. to assist in the completion of these activities. In another example, computing system 102 may be configured to display information such as the status of one or more devices in the medical environment 10, sponge count, blood loss, safety score, procedure information, etc. using one or more displays in the medical environment 10 (e.g., touchscreen 22, monitor 14, or another display).

Returning to FIG. 2, as mentioned above, third medical stage 206 may correspond to one or more activities performed after the medical procedure has completed. These activities may include wheeling a patient out of the operating room, disconnecting medical devices from the patient, cleaning one or more items within the operating room, or other activities. Some of these activities may include one or more tasks that are to be performed. For example, a post-surgical cleaning procedure may include tasks that indicate what medical devices require cleaning and which surfaces to clean on those medical devices, what materials to use to clean particular surfaces and/or objects, how to clean those surfaces and/or objects, how to store objects for subsequent procedures, and other tasks. Some of the activities of third medical stage 206 may include displaying videos, images, checklists, or other information that can be used to guide post-surgical procedures. For example, images, videos, information, and/or checklists may be displayed via monitor 14 (as illustrated in FIG. 1A) to remove patient 12 from medical environment 10 and/or assist medical staff in cleaning medical environment 10.

During the various stages of medical workflow 200 of FIG. 2, different medical content 250 may be presented to some or all of the medical staff within medical environment 10. The particular medical content 250 presented and/or which members of the medical staff that content is presented to may be dependent on the stage of the medical workflow. Medical content 250 may be based on data from content database 168. For example, the medical content stored in content database 168 may correspond to the type of medical procedure being performed. Medical content 250 may include first medical content 252, second medical content 254, and third medical content 256. First medical content 252, second medical content 254, and third medical content 256 may include content relating to various tasks to be performed during first medical stage 202, second medical stage 204, and third medical stage 206, respectively. Accordingly, during first medical stage 202 of medical workflow 200, first medical content 252 may be presented. Likewise, during second medical stage 204, second medical content 254 may be presented. Finally, during third medical stage 206, third medical content 256 may be presented. First medical content 252, second medical content 254, and/or third medical content 256 may be presented using monitor 14, touchscreen monitor 22, or another display (illustrated in FIG. 1A). For example, first medical content 252 may include a checklist of tasks to be performed to prepare an aspect of medical environment 10 for a medical procedure. In another example, second medical content 254 may indicate a current surgical sponge count, blood loss, heart rate, respirations, etc. In another example, third medical content 256 may include a video describing a procedure to clean medical devices, such as medical devices 120 of FIG. 1A, for later use in another medical procedure that may be performed in medical environment 10.

Returning to FIG. 1B, image analysis subsystem 110 may be configured to analyze one or more images of medical environment 10 to determine a stage of a medical procedure, contextual information associated with medical environment 10, or other information. Image analysis subsystem 110 may receive images captured by room cameras 146, camera 152, and/or video camera 140, as described above with reference to FIG. 1A. Room cameras 146, video camera 140, and/or camera 152 may provide captured images/videos to image analysis subsystem 110. For example, room cameras 146 may periodically capture images and/or videos of medical environment 10 (e.g., every second, every 5 seconds, every minute, etc.), which may be provided to image analysis subsystem 110. Image analysis subsystem 110 may be configured to perform one or more pre-processing steps to the received images, which may include image frames extracted from a video. For example, the pre-processing steps may include gray-scaling, cropping, flipping, compressing, etc. the images.

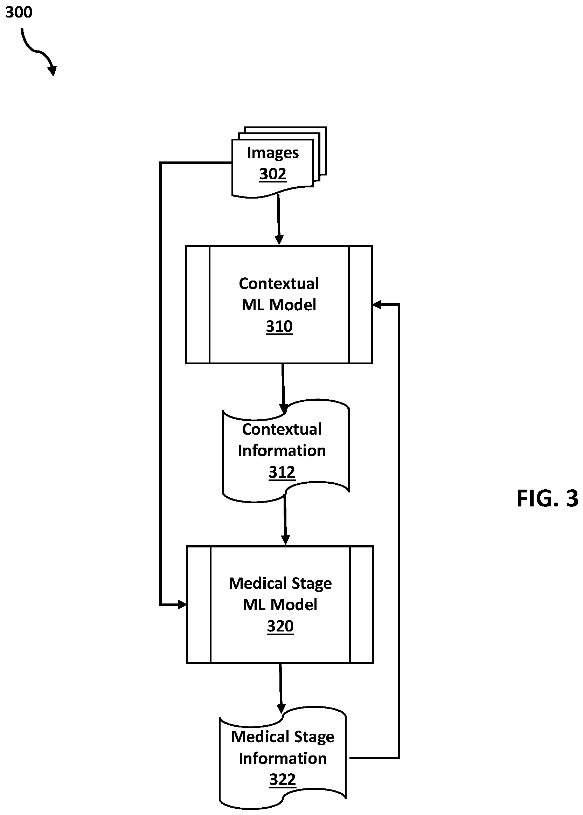

Image analysis subsystem 110 may be configured to analyze the images to extract contextual information associated with the medical procedure and/or identify a medical stage of the medical workflow. For example, image analysis subsystem 110 may be configured to extract contextual information and/or identify the medical stage using one or more machine learning models stored in model database 164. The machine learning models may be arranged as a machine learning pipeline. As an example, with reference to FIG. 3, a machine learning (ML) pipeline 300 may include one or more machine learning models configured to analyze images 302 (e.g., captured by room cameras 146, video camera 140, and/or camera 152) and generate one or more outputs. ML pipeline 300 may be implemented by image analysis subsystem 110. As an example, ML pipeline 300 may include a contextual machine learning model 310 and a medical stage machine learning model 320. Optionally, one or more additional ML models may be included in ML pipeline 300. Alternatively, one or more of the machine learning models 310 and 320 may be omitted or combined. The contextual ML model 310 and medical stage ML model 320 may be executed in parallel or in a sequence (as illustrated in FIG. 3).

It is to be understood that although some aspects are described herein with respect to machine learning models, other prediction models (e.g., statistical models or other analytics models) may be used instead of or in addition to the machine learning models described herein. For example, a statistical model may replace a machine learning model, or vice versa.

Contextual ML model 310 may be configured to extract and output contextual information 312 from input images 302. For example, contextual information 312 may be associated with a medical procedure and/or a medical environment where the medical procedure is being performed, such as medical environment 10 of FIG. 1A. For example, images 302 may be captured using room cameras 146 and may depict medical environment 10.

Medical stage ML model 320 may be configured to output medical stage information 322 based on one or more inputs. For example, medical stage information 322 may indicate a medical stage of the medical workflow identified based on input images 302, as well as, optionally, contextual information 312 extracted using contextual ML model 310. For example, medical stage ML model 320 may determine whether medical workflow 200 of FIG. 2 is currently in a first medical stage 202, second medical stage 204, or third medical stage 206, based on images 302. In another example, medical stage ML model 320 may receive contextual information 312 as input, which may be used to determine medical stage information 322. For example, contextual information 312 may be used to determine whether the medical workflow has progressed from a first stage to a second stage (e.g., from first medical stage 202 to second medical stage 204 of medical workflow 200 shown in FIG. 2).

In some aspect, although contextual ML model 310 is illustrated as analyzing images 302 prior to medical stage ML model 320, the order of the models may be flipped, or, as mentioned above, the models may be executed in parallel. In the instance the models are flipped, medical stage information 322 may be provided as input to contextual ML model 310 such that contextual information 312 may be based on images 302 and/or medical stage information 322. Additionally, other inputs not illustrated in FIG. 3 may be provided to contextual ML model 310 and/or medical stage ML model 320. For example, inputs may include medical images of the patient from previous medical procedures, medical images of other patients that received the same medical procedure being performed and captured in images 302, or other data.

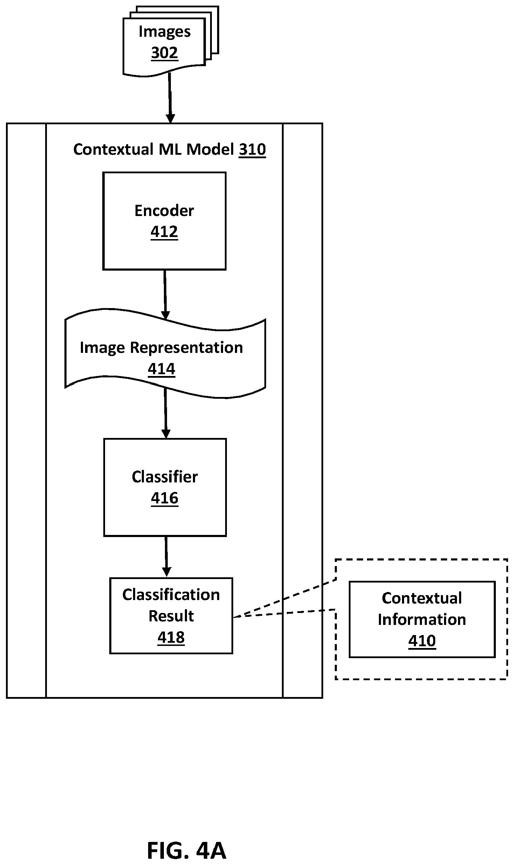

As mentioned above, contextual ML model 310 may be configured to extract contextual information 312 from images 302. Contextual information 312 may describe aspects of a medical procedure, activities performed within a medical environment, objects detected within the medical environment, or other information. For example, contextual information 312 may include a surgical sponge count, information related to blood loss, patient biometric data (e.g., respiratory rate), the number of medical personnel present within medical environment 10, activities performed by some or all of the medical staff, or combinations thereof. An example of contextual ML model 310 is described below with reference to FIG. 4A.

Contextual information 312 may include information associated with cleaning of the medical environment. Contextual ML model 310 may be configured to analyze images 302 to detect cleaning of the medical environment and aspects of the cleaning and generate contextual information 312 that includes information about the detected cleaning. For example, contextual ML model 310 may be configured to detect activity of medical personnel that is associated with cleaning, such as the use of cleaning implements by the medical staff in proximity to objects and/or surfaces to be cleaned. This could include, for example, contextual ML model 310 being trained to detect that one or more medical staff members are grasping a cleaning implement (e.g., a cleaning cloth) and moving the cleaning implement in a cleaning motion in proximity to an object and/or surface to be cleaned (e.g., moving the cloth back and forth in proximity to the surface of a surgical table). Contextual ML model 310 may be configured to track which objects and/or surfaces of objects in the medical environment have been cleaned. For example, contextual ML model 310 may be configured to track the cleaning of tables, surgical lights and control handles, imaging equipment (e.g., a mobile CT scanner), suction regulators, anesthesia carts, compressed gas tanks, floors, walls, and/or any other objects and/or surfaces in the medical environment. The contextual information 312 may include any information associated with detected cleaning activities, such as information that a particular object or surface (e.g., a computer mouse or various surfaces of a surgical table) has or has not been cleaned, an order of cleaning of surfaces of an object (e.g., a surgical table top cleaned before a pedestal of the surgical table), and/or an order of cleaning of objects and/or surfaces (e.g., the surgical table is cleaned before the floors).

FIG. 4A may describe one or more operations of contextual ML model 310. For example, contextual ML model 310 may include an encoder 412 and a classifier 416. Although a single encoder and classifier are illustrated in FIG. 4A, contextual ML model 310 may include any number of encoders and/or classifiers.

Encoder 412 may be implemented as a neural network. The neural network of encoder 412 may include one or more fully connected layers, global and/or max pooling layers, input layers, etc. For example, encoder 412 can be implemented using a variant of a recurrent neural network architecture (RNN) (e.g., a long short-term memory (LSTM) model), a convolutional neural network (CNN) (e.g., ResNet), transformer architectures, other machine learning models, or combinations thereof. In an example, encoder 412 may be implemented using a temporal convolutional network (e.g., a 3D CNN).

Encoder 412 may receive one or more images 302, as described above with reference to FIG. 3, and may generate a corresponding image representation 414 for each received image of images 302. Image representation 414 may be an embedding representing the given image of images 302. The embedding can be a vector representation of the image in a latent space. The embedding may significantly reduce a size and dimension of the original image data. Lower-dimensional embeddings may be used for more efficient downstream processing than processing with the original images 302. The embeddings may be constructed to retain invariant features of the input images 302 while minimizing image-specific characteristics (e.g., imaging angle, resolution, artifacts, etc.). For example, an embedding may be a vector or array of values representing features of a given image from images 302. The quantity of values in the vector may be dependent on the number of features contextual ML model 310 is trained to identify. For example, the embedding may be a vector having 16 values (corresponding to 16 dimensions in the latent space), 128 values (corresponding to 128 dimensions in the latent space), 512 values (corresponding to 512 dimensions in the latent space), or other quantities.

Output image representations 414 from encoder 412 may be provided as input to classifier 416 of contextual ML model 310. Classifier 416 may be a regression classifier, such as a logistic regression classifier, configured to classify an image of images 302 into one or more categories. Each category may be associated with contextual information including but not limited to a particular object, activity, and/or other aspect of a medical procedure. For example, contextual ML model 310 may detect one or more objects present within images 302 based on output image representations 414. Contextual ML model 310 may generate contextual information 312 based on the detected objects. As another example, output image representations 414 may be used to identify activities of medical staff within medical environment 10 (shown in FIG. 1A). Contextual ML model 310 may generate contextual information 312 indicating identified activities of the medical staff based on output image representations 414. Classifier 416 may be configured to output a classification result 418 for each image of images 302. In some examples, different classifiers may be used to determine different classification results 418. Classification result 418 may comprise a score indicating how likely a given image of images 302 represents a particular object, activity, or other item. For example, classifier 416 may determine how likely the given image depicts a particular medical object or one or more of a set of medical objects.

As illustrated in FIG. 4A, classification result 418 may comprise contextual information 410. Contextual information 410 may comprise one or more features of contextual information 312 described above with respect to FIG. 3. For example, contextual information 410 may describe medical objects detected within a medical environment, such as medical environment 10 of FIG. 1A, medical activities detected within medical environment 10, or other contextual information describing aspects of a medical procedure. For example, contextual information 410 may indicate whether contextual ML model 310 detected any objects within images 302. If the instance contextual ML model 310 has detected objects within images 302, contextual information 410 may also indicate what object (or objects) were detected. For example, contextual information 410 may indicate that a medical object such as a surgical sponge, anatomical structure, blood or other bodily fluid loss/volume, etc. was detected within one or more images 302. Contextual information 410 may also indicate whether contextual ML model 310 has detected any medical activities being performed within medical environment 10 (of FIG. 1A) based on images 302. These medical activities may include activities associated with specific personnel of the medical staff (e.g., surgical activities performed by a surgeon, support activities performed by a nurse or technician, etc.). The medical activities may occur over a finite amount of time (e.g., wheeling a patient into the operating room and transferring the patient to the surgical table). Therefore, contextual information 410 may comprise indications of changes in medical environment 10, changes with patient 12, changes within the medical procedure, or other information related to the medical workflow.

Contextual ML model 310, including encoder 412 and classifier 416, may be trained using training data including images depicting various medical objects, medical activities, or other aspects of a medical workflow. The images may be inputted to contextual ML model 310, and contextual ML model 310 may generate a prediction of what objects, activities, etc. those images depict. The predictions may be compared to a ground truth, which may be used to compute a loss (e.g., a cross-entropy loss). The computed loss may be used to adjust one or more hyper-parameters of contextual ML model 310. The process of inputting training data, generating a prediction, comparing the prediction to the ground truth, and computing the loss may be repeated (e.g., iterated) any number of times. Training of contextual ML model 310 may end after a predefined number of training iterations have been performed and/or when contextual ML model 310 produces predictions that meet or exceed a threshold level of accuracy.

Returning to FIG. 3, medical stage ML model 320 may be trained to identify medical stage information from images 302. The medical stage information may indicate a stage of a medical workflow. For example, as described above with reference to FIG. 2, medical workflow 200 may include any number of stages, such as first medical stage 202, second medical stage 204, and third medical stage 206.

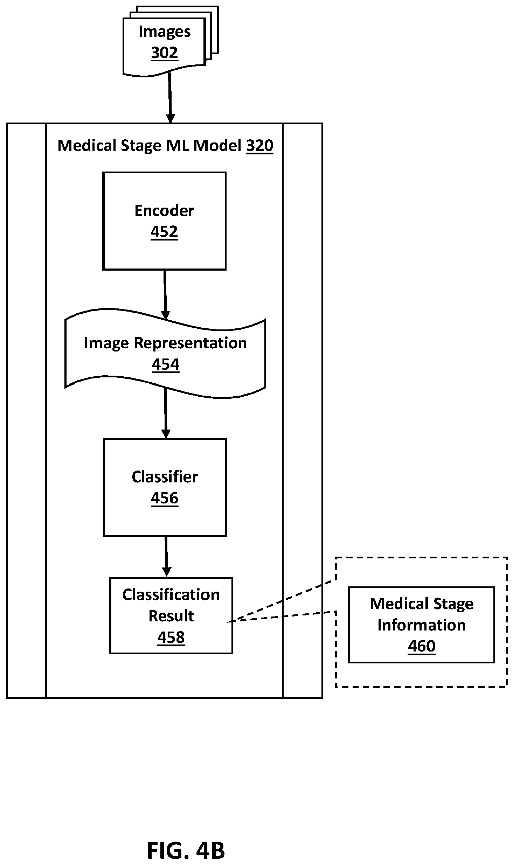

FIG. 4B, illustrates an example of medical stage ML model 320, in accordance with some aspects. Medical stage ML model 320 may receive as input images 302. Medical stage ML model 320 may also receive as input contextual information, such as contextual information 410 of FIG. 4A. Medical stage ML model 320 may include an encoder 452 and a classifier 456. Although a single encoder and classifier are illustrated in FIG. 4B, medical stage ML model 320 may include any number of encoders and/or classifiers. Encoder 452 of FIG. 4B may comprise any one or more features of encoder 412 described above with respect to FIG. 4A.

Encoder 452 may be implemented as a neural network. The neural network of encoder 452 may include one or more fully connected layers, global and/or max pooling layers, input layers, etc. For example, encoder 452 can be implemented using a variant of a recurrent neural network architecture (RNN) (e.g., a long short-term memory (LSTM) model), a convolutional neural network (CNN) (e.g., ResNet), transformer architectures, other machine learning models, or combinations thereof. In an example, encoder 452 may be implemented using a temporal convolutional network (e.g., a 3D CNN).

Encoder 452 may receive one or more images 302 and may generate a corresponding image representation 454 for each of received image of images 302. Image representation 454 may be an embedding representing the given image of images 302. The embedding can be a vector representation of an image in a latent space. The embedding may significantly reduce a size and dimension of the original image data. As mentioned above, lower-dimensional embeddings may be used for more efficient downstream processing than processing with the original images 302. The embeddings may be constructed to retain invariant features of the input images 302 while minimizing image-specific characteristics (e.g., imaging angle, resolution, artifacts, etc.). For example, an embedding may be a vector or array of values representing features of a given image of images 302. The quantity of values in the vector may be dependent on the number of features medical stage ML model 320 is trained to identify. For example, the embedding may be a vector having 16 values (corresponding to 16 dimensions in the latent space), 128 values (corresponding to 128 dimensions in the latent space), 512 values (corresponding to 512 dimensions in the latent space), or other quantities.

The output image representations 454 from encoder 452 may be provided as input to classifier 456 of medical stage ML model 320. Classifier 456 may be a regression classifier, such as a logistic regression classifier, configured to classify an image of images 302 into one or more categories. Each category may be associated with a particular medical stage of a medical workflow, such as first medical stage 202, second medical stage 204, third medical stage 206, etc. of medical workflow 200 illustrated in FIG. 2. Classifier 456 may be configured to output a classification result 458 for each image of images 302. In some examples, different classifiers may be used to determine different classification results 458. Classification result 458 may comprise a score indicating how likely a given image of images 302 represents a particular medical stage. For example, classifier 456 may determine how likely the given image depicts first medical stage 202 of medical workflow 200.

As illustrated in FIG. 4B, classification result 458 may comprise medical stage information 460. Medical stage information 460 may comprise any one or more features of medical stage information 322 described above with respect to FIG. 3. For example, medical stage information 460 may indicate whether medical stage ML model 320 determined a medical stage of a medical workflow based on images 302. In the instance medical stage ML model 320 determined a medical stage based on images 302, medical stage information 460 may also indicate what medical stage was identified. For example, medical stage information 460 may indicate that medical workflow 200 of FIG. 2 is currently in first medical stage 202. Classification result 458 may also be determined based at least in part on contextual information 410 (shown in FIG. 4A). For example, the medical stage may be determined based on medical objects and/or activities detected within the medical environment, such as medical environment 10 of FIG. 1A, based on images 302. As mentioned above, different objects and/or activities may be associated with different stages of the medical workflow. Therefore, contextual information 410 extracted by contextual ML model 310 of FIG. 4A may be used by medical stage ML model 320 to determine medical stage information 460.

Classification result 458 may also include an indication of a subsequent medical stage. The subsequent medical stage may correspond to the known stage of the medical workflow that follows the current medical stage. For example, medical stage ML model 320 may determine that medical workflow 200 is currently in first stage 202. Thus, classification result 458 may indicate the current stage (first medical stage 202 of medical workflow 200), as well as a following medical stage of the medical workflow (e.g., second medical stage 204).

Medical stage ML model 320, including encoder 452 and classifier 456, may be trained using training data including images depicting various medical stages of a medical workflow. The images may be inputted to medical stage ML model 320, and medical stage ML model 320 may generate a prediction of what medical stages those images depict. The predictions may be compared to a ground truth, which may be used to compute a loss (e.g., a cross-entropy loss). The computed loss may be used to adjust one or more hyper-parameters of medical stage ML model 320. The process of inputting training data, generating a prediction, comparing the prediction to the ground truth, and computing the loss may be repeated (e.g., iterated) any number of times. Training of medical stage ML model 320 may end after a predefined number of training iterations have been performed and/or when medical stage ML model 320 produces predictions that meet or exceed a threshold level of accuracy.

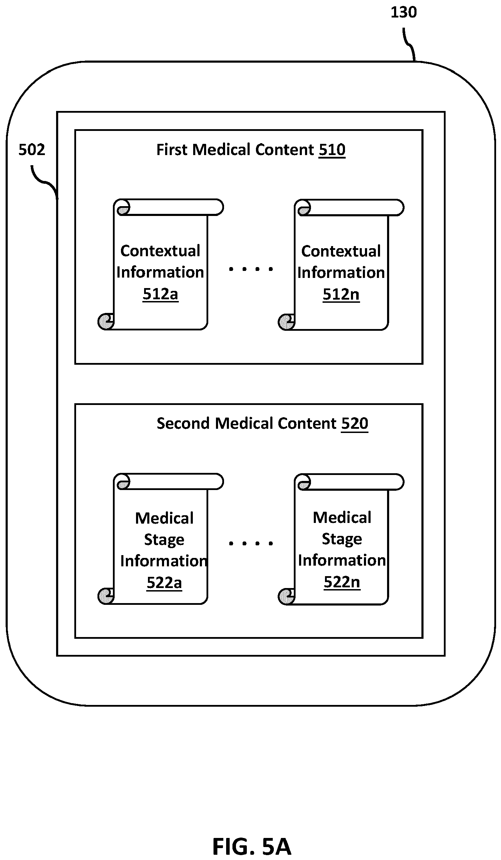

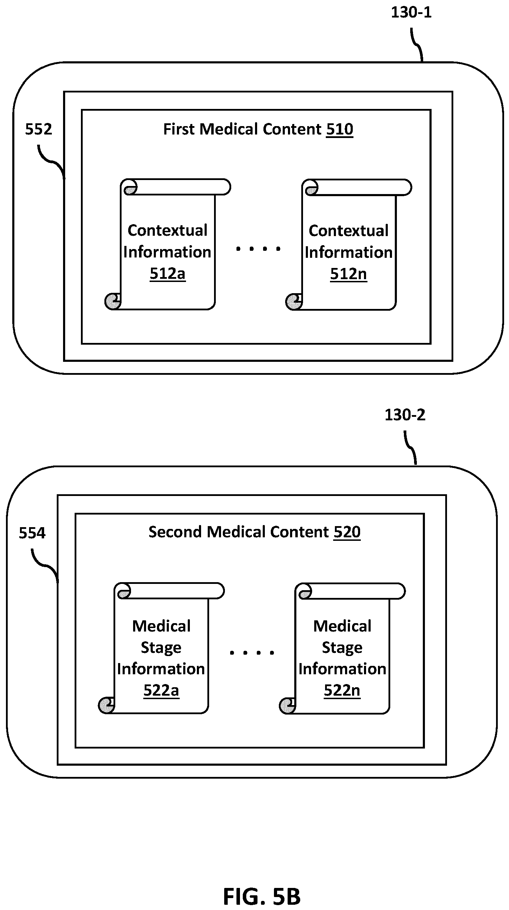

Medical content presented to medical staff within a medical environment (e.g., medical environment 10 illustrated in FIG. 1A) may be selected (e.g., from previously generated content stored in a database, such as content database 168) and/or generated based on the extracted contextual information 410 and/or the identified medical stage information 460. For example, the medical content may be generated based on one or more checklists and/or one or more videos to be presented to the medical staff. With reference to FIG. 5A, a display 502 of client device 130 may present first medical content 510 and second medical content 520. First medical content 510 and second medical content 520 may be presented via a user interface of client device 130 rendered on display 502. As another example, with reference to FIG. 5B, first medical content 510 may be displayed on a first display 552 of a first client device 130-1, and second medical content 520 may be displayed on a second display 554 of a second client device 130-2.

A report describing the medical workflow may be presented to a user (e.g., a surgeon, medical staff, etc.) via first client device 130-1, second client device 130-2, monitor 14, touchscreen 22, and/or another device. The report may include first medical content 510, second medical content 520, other content, and/or combinations thereof. The report may also include the contextual information, the identified stage, the subsequent stage, and/or other information. For example, a report may be generated that describes discrepancies found between the machine learning models' determinations (e.g., the contextual information) and the medical staff's findings.

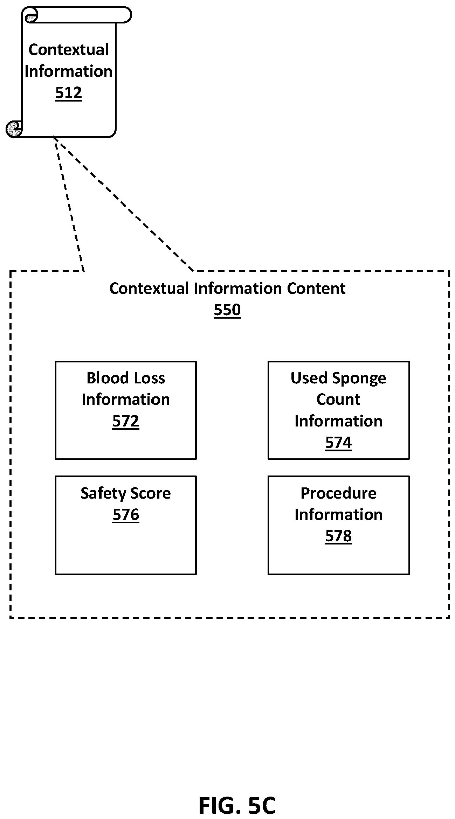

As illustrated in FIGS. 5A and 5B, first medical content 510 may include contextual information 512a to contextual information 512n (collectively referred to herein as contextual information 512). Each of contextual information 512 may depict the same or different information. As an example, with reference to FIG. 5C, contextual information 512 may comprise one or more forms of contextual information content 550 including blood loss information 572, used sponge count information 574, a safety score 576, procedure information 578, or other information not explicitly illustrated in FIG. 5C.

Blood loss information 572 may indicate an estimated amount of blood lost during the medical procedure. Blood loss information 572 may be determined based on contextual ML model 310 (shown in FIG. 3). For example, contextual ML model 310 may determine how much blood has been lost during surgery based on an analysis of images of medical environment 10 (shown in FIG. 1A). Contextual ML model 310 may also receive inputs from other medical devices 120. For example, a suction device used to remove blood or other fluids from an anatomical region of patient 12 may be configured to provide blood loss information 572 to contextual ML model 310 (e.g., via network 170) indicating an amount of blood (or other fluids) collected.

Used sponge count information 574 may indicate a number of surgical sponges used during surgery. For example, contextual ML model 310 may analyze images of medical environment 10 to determine when a new surgical sponge is obtained, a used surgical sponge is disposed, etc. Additionally, contextual ML model 310 may analyze audio (e.g., captured by microphone 16 shown in FIG. 1A) detected within medical environment 10. For example, voice commands or speech may be dictated within medical environment 10 and indicate a quantity of used sponges, and contextual ML model 310 may detect and analyze the voice commands or speech to determine, record, and/or display the used sponge count information 574.

Safety score 576 may be or include a numerical value (e.g., ranging from 0 to 100) indicating a quantified safety of patient 12. Safety score 576 may be computed based on inputs from medical devices 120 and/or sensors within medical environment 10. For example, an entryway to medical environment 10 may include one or more sensors that detect when the entryway has been opened or when someone has passed through the entryway. The number of times that the entryway has been opened or someone has passed through the entryway may be used in determining safety score 576, where a number of entries less than a threshold number of entries may yield a higher safety score than a number of entries greater than the threshold number of entries. As another example, one or more temperature sensors disposed in medical environment 10 may be configured to monitor the temperature of medical environment 10. The monitored temperature may also be used in determining safety score 576, where a monitored temperature within a threshold temperature range may yield a higher safety score 576 than a monitored temperature outside of the threshold temperature range.

Safety score 576 may be associated with effectiveness of cleaning of the medical environment, which can impact patient safety (e.g., by affecting infection risk to a patient). Safety score 576 may correspond with how effectively the medical environment 10 was cleaned according to predetermined rules. For example, the safety score 576 may be based on a proportion of the surfaces and/or objects in the medical environment 10 that were cleaned (such as determined by contextual ML model 310 of FIG. 3) versus the surfaces and/or objects that should have been cleaned according to predetermined cleaning rules. Additionally, or alternatively, and/or the safety score 576 may be based on an order in which objects were cleaned (e.g., a table top of a surgical table is cleaned before a pedestal of the surgical table and/or a table is cleaned before a floor). The safety score 576 may be based on the relative importance of cleaning of different objects and/or surfaces, such as whether a given object and/or surface is high touch or low touch. For example, the failure to clean a surgical tabletop (e.g., a high touch surface) may result in a relatively lower safety score 576 than a failure to clean a floor (e.g., a low touch surface). The predetermined cleaning rules can be associated with, for example, hospital guidelines and/or recommended best practices. Different sets of predetermined cleaning rules may be associated with different stages of cleaning. For example, a different set of cleaning rules may be used for determining the effectiveness of scheduled deep cleaning than for determining the effectiveness of a regular cleaning. As such, the safety score 576 may be associated with a given medical stage, such as determined by medical stage ML model 320.

Optionally, multiple different safety scores 576 may be determined. The different safety scores 576 may include different types of safety scores 576. For example, one safety score may be based on a number of entries into a medical environment and another safety score may be based on an effectiveness of cleaning of the medical environment. The different safety scores 576 may include the same type of safety scores 576 but for different medical stages. For example, one safety score may be associated with pre-operative cleaning and another safety score may be associated with post-operative cleaning. The different safety scores 576 may be displayed simultaneously or during different medical stages. For example, a safety score associated with a number of entries into the medical environment may be displayed during a medical procedure and a safety score associated with an effectiveness of cleaning may be displayed after completion of a medical procedure. Different safety scores can be provided simultaneously. For example, a post-operative report may include a safety score associated with a number of entries into the medical environment and a safety score associated with an effectiveness of pre-operative cleaning. A safety score 576 associated with effectiveness of cleaning can be used to improve the cleaning (e.g., cleaners can continue cleaning until the safety score 576 reaches a desired value) and/or can be used for post-cleaning assessment and/or correlation with patient outcomes.

Procedure information 578 may indicate information associated with medical stages of the medical workflow. For example, procedure information 578 may indicate that medical workflow 200 of FIG. 2 is currently in the first medical stage 202. Procedure information 578 may instead or additionally indicate information associated with phases of a medical procedure being performed. For example, procedure information 578 may describe a current surgical phase of a surgical procedure (e.g., preoperative, perioperative, intraoperative, postoperative, and post-discharge).

Contextual information 512 may be presented using various devices within medical environment 10 (shown in FIG. 1A). For example, used sponge count information 574 may be presented on one display (e.g., monitor 14), while procedure information 578 may be presented on another display (e.g., touchscreen 22).

Returning to FIGS. 5A and 5B, second medical content 520 may include medical stage information 522a to medical stage information 522n (collectively referred to herein as medical stage information 522). Medical stage information 522 may include images, videos, checklists, descriptions, or other information describing a current medical stage, a previous medical stage, and/or a subsequent medical stage. As mentioned above, a variety of tasks may need to be performed during each medical stage. For example, some of the medical staff may be capturing images of a patient, while concurrently, some of the medical staff may be preparing a mixture for the surgical procedure. The types of information presented for these tasks may also vary. For example, a first set of medical stage information may be presented to one subset of the medical staff, while a second set of medical stage information may be presented to another subset of the medical staff. Continuing the previous example, the first set of medical stage information may include one or more videos detailing what images to capture and/or how to capture those images. The second set of medical stage information may include a checklist of steps to be performed to correctly prepare the mixture.

Medical stage information 522 may include information associated with cleaning of the medical environment. For example, medical stage information 522 may include a checklist indicating the objects and/or surface to be cleaned and/or the status of cleaning of the objects and/or surfaces (e.g., whether a given object or surface has been cleaned).

The particular types of medical stage information 522, and to whom that medical stage information is to be presented, may be determined based on images (e.g., images 302) captured during the medical workflow (e.g., medical workflow 200 illustrated in FIG. 2). For example, during medical workflow 200, one or more images may be captured using room cameras 146, camera 152, and/or video camera 140 of FIG. 1A. The types of medical stage information 522 and to whom it may be presented may instead or additionally be determined based on contextual information 512 (shown in FIG. 5C), configurations of medical environment 10 (shown in FIG. 1A), other information, or combinations thereof.

Returning to FIG. 1B, content rendering subsystem 112 may be configured to execute one or more ML models (e.g., medical stage ML model 320) to identify a stage of the medical workflow based on images captured during medical workflow 200 (illustrated in FIG. 2). For example, as mentioned above, the stage of medical workflow 200 may be determined based on images captured by room cameras 146, camera 152, and/or video camera 140 of FIG. 1A. Content rendering subsystem 112 may instead or additionally be configured to select medical stage information 522 to be presented on a display based on the identified stage of the medical workflow. Content rendering subsystem 112 may also be configured to receive contextual information 512 (shown in FIG. 5C) to identify the stage (e.g., using one or more ML models). For example, medical stage information 522 may be selected based on the identified current stage, a preceding stage, and/or a subsequent stage. The preceding stage may include the stage immediately preceding the identified stage. For example, if the current stage of medical workflow 200 is determined to be the second medical stage 204, then the preceding stage may be the first medical stage 202. Alternatively, the preceding stage may correspond to one or more earlier occurring stages of medical workflow 200. For example, if the current stage of medical workflow 200 is determined to be the third medical stage 206, then the preceding stage may be the first medical stage 202 and/or the second medical stage 204. The subsequent stage may include the stage immediately following the identified stage. For example, if the current stage of medical workflow 200 is determined to be the second medical stage 204, then the subsequent stage may be the third medical stage 206. Alternatively, the subsequent stage may correspond to one or more later occurring stages of medical workflow 200. For example, if the current stage of medical workflow 200 is determined to be the first medical stage 202, then the subsequent stage may be second medical stage 204 and/or the third medical stage 206.

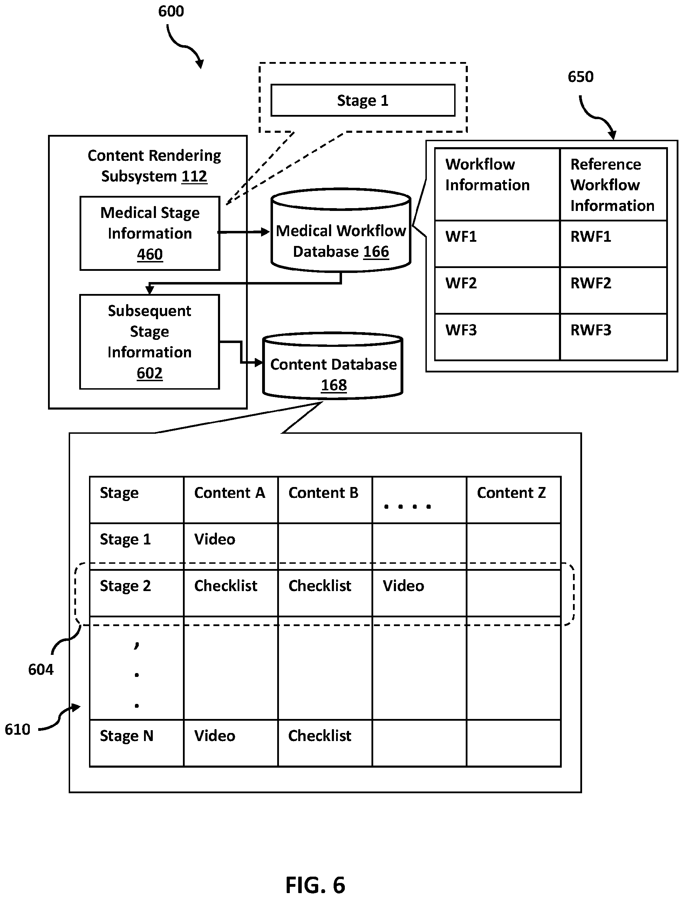

FIG. 6 illustrates an example system 600 that includes content rendering subsystem 112. Content rendering subsystem 112 may be configured to obtain medical stage information 460, previously illustrated in FIG. 4B. Medical stage information 460 may indicate a current stage of a medical workflow, such as medical workflow 200 of FIG. 2. As mentioned above, medical stage information 460 may be determined based on images captured of the medical environment (e.g., by room cameras 146, camera 152, and/or video camera 140 illustrated in FIG. 1A), contextual information 512 extracted from images 302 (shown in FIG. 4A), other information, or combinations thereof.

As shown in FIG. 6, content rendering subsystem 112 may access medical workflow database 166 to identify subsequent stage information 602 of the medical workflow 200. For example, if medical stage information 460 indicates that the current stage is "Stage 1," subsequent stage information 602 may indicate "Stage 2." Medical stage information 460 may be used to generate content associated with a subsequent medical stage of medical workflow 200. For example, content rendering subsystem 112 may generate an audio message indicating tasks and/or activities associated with the subsequent stage of medical workflow 200 based on subsequent stage information 602.

Medical workflow database 166 may include a data structure 650. Data structure 650 may store medical workflow information (e.g., WF1, WF2, and WF3) and reference medical workflow information (e.g., RWF1, RWF2, and RWF3). Medical workflow information WF1, WF2, WF3 may be associated with a first medical workflow, a second medical workflow, and a third medical workflow, respectively. For example, information related to different medical procedures may correspond with one or more different medical workflows. Each medical workflow may include one or more stages, which may be indicated by the respective medical workflow information. For example, medical workflow information WF1 may relate to medical workflow 200 of FIG. 2 and may include information related at least to first medical stage 202, second medical stage 204, and third medical stage 206.

For each medical workflow, data structure 650 may store reference medical workflow information RWF 1, RWF2, RWF3 respectively corresponding to WF1, WF2, WF3. Reference medical workflow information RWF 1, RWF2, RWF3 may indicate efficiency parameters. The efficiency parameters may include, for example, benchmark times to complete one or more stages of the medical workflow, a number of medical staff allocated for the medical workflow and/or for particular stages of the medical workflow, etc. The efficiency parameters of a medical workflow may be determined from data related to previously recorded performances of the medical workflow. For example, the amount of time allotted for a given medical workflow may be determined based on an average amount of time expended by one or more other surgeons in completing the given medical workflow.

Each workflow WF1, WF2, WF3 may indicate a total surgical time of a surgical procedure, an operating room turnaround time, staff efficiencies, a surgical procedure efficiency score, or other information gathered during use of the medical environment. The total surgical time may indicate the amount of time that a given surgical procedure takes to complete. The operating room turnaround time may indicate the amount of time that it takes the medical staff to clean and prepare the medical environment (e.g., an operating room) for a subsequent surgical procedure that may occur in the medical environment. The staff efficiencies may indicate the efficiency of some or all of the medical staff during the given surgical procedure. The surgical procedure efficiency score may indicate the overall efficiency of the current surgical procedure in comparison to a reference surgical procedure (e.g., based on the reference workflow information).

Content rendering subsystem 112 may be configured to determine which content to present based on the current stage, the subsequent stage (e.g., subsequent stage information 602), the preceding stage, or combinations thereof. For example, content rendering subsystem 112 may access a data structure 610 (e.g., stored in content database 168) and may identify row 604 in data structure 610 that corresponds to subsequent stage information 602 (e.g., "Stage 2"). Data structure 610 may store data indicating which content can be presented for a particular medical stage. Some stages may have a single content item to present (e.g., Stage 1 includes a video to be presented), whereas other stages may indicate multiple content items to be presented (e.g., Stage 2 includes two checklists and a video to be presented). In the instance multiple content items are to be presented, these content items may be presented on the same display or different displays. For example, some content items may be presented using monitor 14, while other content items may be presented using touchscreen monitor 22 of FIG. 1A. Determining which displays to use to present particular content may be based on the type of content to be presented, the proximity of the display device to a particular medical device, the location of the display within the medical environment, the respective positions of medical staff within the medical environment, the respective roles and/or activities performed by medical staff, a system configuration (e.g., medical device 120 and/or additional monitor 14 configuration) specific to medical environment 10, etc.

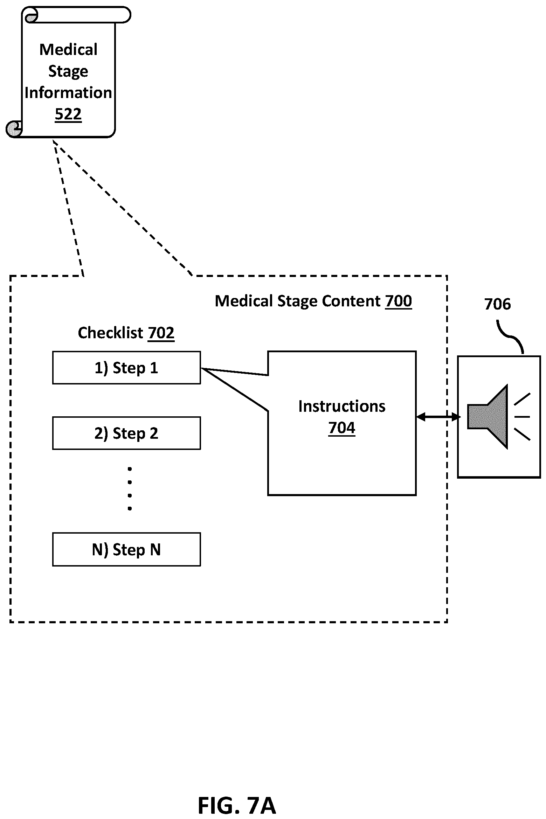

As the medical staff proceeds through the different tasks associated with the different stages of the medical workflow, the content may dynamically update. For example, as seen with reference to FIGS. 7A-7B, medical stage information 522 may include medical stage content 700. Medical stage content 700 may include a checklist 702 comprising A steps (e.g., step 1, step 2, ..., step N). An exemplary checklist 702 may provide guidance for a cleaning procedure. For example, the N steps could indicate the objects and/or surfaces to be cleaned and/or an order of the objects and/or surfaces to be cleaned. As the steps of checklist 702 are completed, a graphical indication of completion of completed steps may be provided. For example, a completed step may be "checked off." Additionally, or alternatively, the content presented may be modified as the step of checklist 702 are completed. For example, as shown in FIG. 7A, the presented content may be related to step 1 of checklist 702. As shown, the medical content related to step 1 may include instructions 704 that may be presented to one or more personnel (e.g., medical staff) in the medical environment. Instructions 704 may detail how to complete Step 1 (e.g., how to measure materials for preparing a mixture or how to clean a given object and/or surface). Instructions 704 may be graphically presented within a user interface rendered by a display of client device 130, medical device 120, monitor 14, touchscreen 22, or another display device in medical environment 10 described above. In addition to or instead of instructions 704, an audio message 706 may be output to the medical staff to assist in completing the step. For example, audio message 706 may be output via speakers 118 shown in FIG. 1A. Audio message 706 may include a text-to-speech transformation of instructions 704. One or more videos (e.g., instructional videos) may be presented via monitor 14 and/or touchscreen 22 in addition to or instead of instructions 704 and/or audio message 706. For example, the videos may describe how to perform a particular step. The content (e.g., checklist 702, instructions 704, audio message 706, etc.) may be stored in content database 168 (illustrated at least in FIG. 6). For example, the columns of data structure 610 in FIG. 6 may store location data indicating the location of data representing the content within content database 168 or within another data repository. In one example, the location data may include a hyperlink or pointer to the content to be presented. The content may be identified and/or generated based on contextual information, medical stage information, and/or other information associated with the medical workflow. For example, one or more checklists 702 associated with medical workflow 200 (shown in FIG. 2), one or more videos associated with medical workflow 200, and/or other content may be identified. Content may be generated based on the identified checklists 702 (e.g., second medical content 520 of FIGS. 5A-5B), one or more videos, and/or other content, which may be presented to the users within medical environment 10 (shown in FIG. 1A).

Audio message 706 may also indicate tasks and/or actions to be performed by a user during the subsequent medical stage. Audio message 706 describing the tasks and/or actions to be performed during the subsequent medical stage may be stored, for example, in content database 168 (illustrated at least in FIG. 6). Content rendering subsystem 112 may be configured to generate audio message 706 based on information associated with a subsequent stage of the medical workflow. For example, content rendering subsystem 112 may determine a subsequent medical stage based on medical stage information 460 (shown in FIG. 4B) indicating a current medical stage of the medical workflow, and may generate audio message 706 based on medical stage information 460 associated with the subsequent medical stage.

As step 1 is being executed, images depicting the medical environment may continue to be received at computing system 102. For example, room cameras 146 shown in FIG. 1A may capture images (e.g., images 302) depicting some or all of medical environment 10. These images may be provided to image analysis subsystem 110 (shown in FIG. 1B). Image analysis subsystem 110 may determine, based on the captured images, whether the current step has been completed. For example, the images may indicate activities of the medical staff within medical environment 10. Based on these activities, image analysis subsystem 110 may be configured to determine whether the current step being executed (e.g., step 1) and/or the medical stage associated with the current step, has been completed.

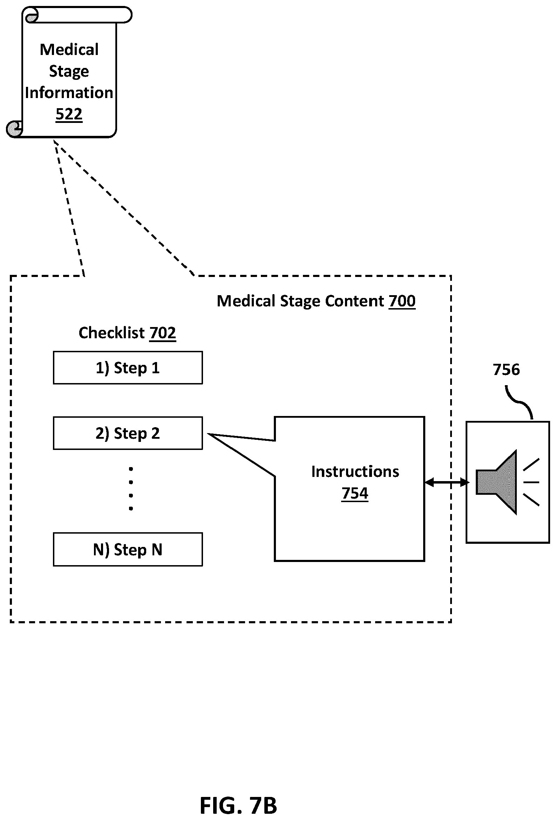

Content rendering subsystem 112 may be configured to present updated medical content to reflect the next step. For example, with reference to FIG. 7B, following a determination by image analysis subsystem 110 that step 1 of checklist 702 has been completed, image analysis subsystem 110 may determine the subsequent step in checklist 702. For example, in checklist 702, the subsequent step may be step 2 illustrated in FIG. 7B. Content rendering subsystem 112 may be configured to retrieve and output medical stage content 700 associated with the subsequent step (e.g., step 2). For example, at Step 2, instructions 754 and/or audio message 756 may be output to the medical personnel in the medical environment.

Content rendering subsystem 112 may also be configured to notify one or more medical staff of a task being performed out of order or before another task has been completed. For example, for a given medical stage, a first step associated with a current task and a second step associated with a subsequent task may be identified. The first and second steps may be identified from a plurality of steps associated with the given stage. For example, with reference to FIGS. 7A and 7B, a particular medical stage identified in medical stage information 522 may include checklist 702 comprising steps 1-N. If it is determined that one or more medical staff members have begun performing tasks associated with the second step before the first step has been completed (e.g., based on images 302 shown in FIG. 3), content rendering subsystem 112 may generate and output a notification to the medical staff. For example, the notification may be an audible alert, a visual alert, and/or a haptic alert. For example, the alert may be an audio message output via speakers 118 of FIG. 1A, a visual message presented on monitor 14 and/or touchscreen monitor 22, a haptic alert, or another format, or a combination thereof. The notification may be directed to a display and/or audio device located proximate to the medical staff members determined to be preemptively performing the tasks associated with the second step.

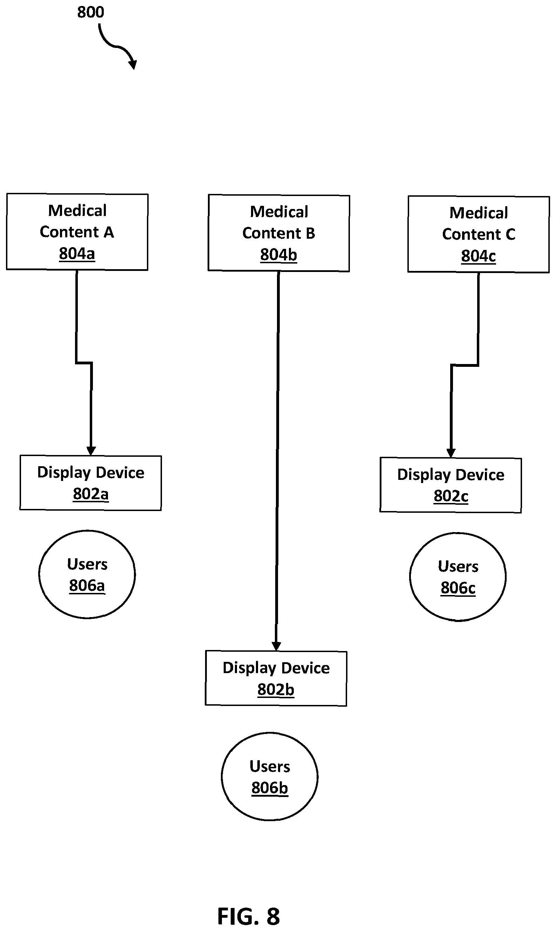

As mentioned above, content rendering subsystem 112 (shown in FIG. 1B) may be configured to determine the content to be presented on the various displays within the medical environment. The displays used to present content may be selected based on the type of content to be presented, a proximity of the display device to a given medical device, the location of those displays within the medical environment, the respective positions of medical staff within the medical environment, the respective roles and/or activities performed by medical staff, a system configuration (e.g., medical device 120 and/or additional monitor 14 configuration) specific to medical environment 10, etc. For example, with reference to FIG. 8, a system 800 may include display devices 802a-802c. Additional or fewer display devices may be included in system 800. Display devices 802a-802c may correspond to one or more client devices 130, medical devices 120, monitor 14, touchscreen monitor 22, or other devices located in medical environment 10. One or more of display devices 802a-802c may be selected to present medical content (e.g., first medical content 510 and/or second medical content 520 shown in FIGS. 5A-5B). The selected one or more of display devices 802a-802c may be sent the medical content to be presented. For example, content rendering subsystem 112 (shown in FIG. 1B) may select one or more of display devices 802a-802c to present second medical content 520 (shown in FIGS. 5A-5B) to one or more users within medical environment 10, and may send second medical content 520 to the selected one or more of display devices 802a-802c for display.

User groups 806a-806c may be located proximate display devices 802a-802c, respectively. User groups 806a-806c may be identified as being proximate to display devices 802a-802c based on contextual information (e.g., contextual information 410 described above with respect to FIG. 4A). For example, one or more relevant medical staff may be identified based on their proximity to display devices 802a-802c. The relevant medical staff may be determined from the total medical staff present in the medical environment (e.g., medical environment 10 in FIG. 1A). For example, image analysis subsystem 110 (shown in FIG. 1B) may be configured to identify the one or more relevant medical staff included in each of user groups 806a-806c by identifying the respective roles and/or activities performed by those medical staff. Based on the identified relevant medical staff, content rendering subsystem 112 may be configured to direct content 804a-804c to one of display devices 802a-802c. For example, content rendering subsystem 112 may direct medical content 804a to display device 802a for presentation, medical content 804b to display device 802b for presentation, and/or medical content 804c to display device 802c for presentation. Medical content 804a may be presented to one or more of the members (e.g., the relevant medical staff) of user group 806a using display device 802a. Similarly, medical content 804b and 804c may be presented to one or more of the members of user groups 806b and 806c using display devices 802b and 802c, respectively.

A given member's role in the medical workflow may be determined based on activities performed by that member. As mentioned above with reference to FIG. 1A, room cameras 146, camera 152, and/or video camera 140 may capture images of medical environment 10, which may be analyzed to determine the activities of the given member. These activities may include, but are not limited to, wheeling patient 12 into medical environment 10, wheeling patient 12 out of medical environment 10, preparing a mixture, capturing a medical image, etc. The activities may also include detected user idleness. User idleness may be detected using image analysis subsystem 110 to determine that the images of medical environment 10 include a person that is not moving (e.g., for a predetermined duration of time) and/or not performing an activity associated with the medical workflow (e.g., medical workflow 200 of FIG. 2). Idleness may also be detected based on increased activity of other personnel within medical environment 10 in comparison to the idle person. For example, a given member may perform tasks that another member was expected to perform, which may indicate a lack of activity for the latter member.

An efficiency score for the medical procedure (e.g., a surgical efficiency) may be computed based on detected activities. For example, the efficiency score may be computed on a medical procedure level, a medical staff level, a medical stage level, or at other granularities. An efficiency score may be calculated based on a total time that a given medical procedure takes to complete, an amount (e.g., percentage) of idleness detected within the medical environment, a comparison between the total time and an expected time for completion of the given surgical procedure, or other information. An efficiency score may be calculated based on detected cleaning activity, such as an amount of time taken to clean and/or the adherence of the cleaning to predetermined rules associated with cleaning efficiency. To evaluate the efficiency of a surgery and therefore compute the efficiency score, a video recording of the medical procedure can be analyzed using one or more machine learning models (e.g., contextual ML model 310 and/or medical stage ML model 320 of FIG. 3). These machine learning models may be configured to generate a report based on the analyzed video. The report may describe the contextual information, such as the detected activities, efficiency score, total time, etc. The report may instead or additionally describe the presented content, an identified stage, a subsequent stage, and/or other information. For example, a report may be generated that describes discrepancies found between a machine learning models' determinations (e.g., the contextual information) and findings of the medical staff.

As an example, the report may include:
1) A total amount of time required to complete the medical procedure;
2) A comparison of the total amount of time to an average amount of time required for the same surgeon and/or one or more different surgeons to complete the same or similar medical procedure;
3) A total number of people performing and/or associated with the medical procedure, including automatic classification of the medical staff (classifications may include, e.g., surgeon, assisting surgeon, nurse, anesthesiologist, etc.);
4) An amount of time expended during each medical stage of the medical workflow;
5) A comparison of the amount of time expended during each stage to an average amount of time expended during a corresponding medical stage of a same or similar medical workflow;
6) A number of people (e.g., of the medical staff) involved in each medical stage of the medical workflow and their automatically identified roles;
7) A relative amount of time each member of the medical staff was actively involved in some or all of the medical stages of the medical workflow;
8) A relative amount of time each member of the medical staff was determined to be idle during some or all of the medical stages of the medical workflow; and/or
9) Any adverse medical events detected during the medical workflow, indicated in a list for each medical stage of the medical workflow.

In addition, the machine learning models may be configured to generate a video summary. The video summary may include an identification of the stages of the medical workflow. The video summary may be integrated with the medical report, which may enable a reviewer to easily navigate to a specific time during the medical workflow and watch a corresponding video summary (e.g., a video snippet). The video summary may instead or additionally be generated for each member of the medical staff, particularly corresponding to when each member was actively involved in the various stages of the medical workflow.

A report for the medical workflow may be generated describing the presented content, the contextual information, the identified stage, the subsequent stage, and/or other information. For example, a report may be generated that describes discrepancies found between the machine learning models' determinations (e.g., the contextual information) and the medical staff's findings. The report may include first medical content associated with the contextual information and second medical content associated with the subsequent stage of the medical workflow.

As described above, the efficiency score may be computed in a number of different ways, each of which may be aggregated together to obtain an overall efficiency score for the medical workflow. Alternatively, one or more of the individual efficiency scores may be used to describe the efficiency of a given medical workflow.

In one example, the machine learning models implemented by computing system 102, room cameras 146, medical devices 120, and/or client devices 130 (shown in FIG. 1B) may be configured to compute an efficiency score based on an amount of time that a user waits to obtain a requested tool or equipment. For example, a surgeon may request a tool or equipment for use during a surgical procedure. By monitoring the activities of all personnel within medical environment 10, one or more of the machine learning models may identify when the surgeon requested tool or equipment, when the surgeon has received the requested tool or equipment, and/or a lapsed amount of time between those two events. The efficiency score may be computed based on the lapsed amount of time between those two events. In this example, decreasing the lapsed amount of time between when the surgeon requests the tool/equipment and when the surgeon receives the tool/equipment may increase the efficiency score.

In another example, an efficiency score may be computed based on a comparison with reference medical workflow information. For example, the efficiency score may be compared with efficiency scores and/or other benchmark parameters stored in data structure 650 of FIG. 6. Reference workflow information RWF1, RWF2, RWF3 may indicate a total surgical time of a surgical procedure, an operating room turnaround time, staff efficiencies, a surgical procedure efficiency score, or other information. The total surgical time may indicate an average expected amount of time required to complete a given surgical procedure. The operating room turnaround time may indicate an expected amount of time required for the medical staff to clean and prepare the medical environment (e.g., an operating room) for a subsequent surgical procedure that may be performed in the medical environment. The staff efficiencies may indicate an expected efficiency of some or all of the medical staff during a given surgical procedure. The surgical procedure efficiency score may indicate the efficiency of the current surgical procedure compared to a reference surgical procedure. Reference workflow information may be determined based on medical workflows previously performed by one or more senior medical staff members. The reference workflow information may be stored in medical workflow database 166. Additionally, medical workflow database 166 may include multiple reference workflows (e.g., RWF1, RWF2, RWF3), each reference workflow associated with a particular medical procedure, medical staff, and/or surgeon.

As another example, an efficiency score for the medical workflow may be computed based on an amount of time that one or more members of the medical staff were idle during the stages of the medical workflow. Based on images captured by room cameras 146, camera 152, and/or video camera 140 (shown in FIG. 1A), the activities or idleness of the total medical staff may be determined. The efficiency score may be computed on an individual medical staff member level. For example, the amounts of time that each individual medical staff member is idle and active may be used to determine how efficient that medical staff member was during the medical workflow. Each medical staff member's efficiency score may be analyzed to determine whether the number of medical staff allocated for a given medical workflow should be increased, decreased, or kept the same. By adjusting the number of medical staff provided for each medical workflow, allocation of medical staff can be optimized.

As described above, the efficiency score may be used to optimize aspects of the medical workflow. Optimizing the medical workflow may include: (i) notifying the team of an area in the medical environment prone to collision and optimizing the layout of objects in the medical environment based on the notification before surgery, (ii) providing training to medical staff, (iii) increasing the number of medical staff allocated for a medical procedure (e.g., in the instance the efficiency score determined for each of the currently allocated medical staff meets or exceeds a threshold efficiency score, such as greater than 75% efficiency, greater than 85% efficiency, greater than 90% efficiency, etc.), (iv) adjusting a location of medical devices 120 or other equipment within medical environment 10 (shown in FIG. 1A) based on a total amount of detected movement of the medical staff, (v) notifying medical staff of pre-surgical tasks to be performed, such as maintaining proper hygiene and maintaining proper distance from equipment, surgical table, sterile zone, etc., (vi) notifying one or more circulating nurses about the state of the surgical procedure and/or any outstanding requests during the surgical procedure such that the number of circulating nurse visits can be minimized to allow circulating nurses to complete their assigned tasks, and/or (vii) producing a medical report indicating how future medical procedures may be optimized.

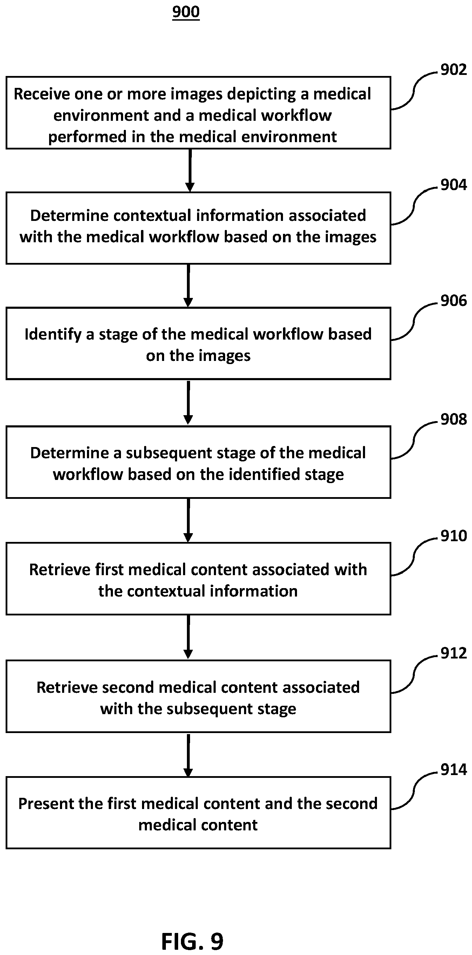

FIG. 9 illustrates a flowchart of an example process 900, according to some aspects. Process 900 may begin at step 902. At step 902, one or more images depicting a medical environment and a medical workflow performed in the medical environment may be received. The medical environment may correspond to an operating room. The medical workflow may correspond to the various stages associated with a medical procedure. For example, medical workflow 200 shown in FIG. 2 may include first medical stage 202, second medical stage 204, and third medical stage 206. The medical workflow stages for a surgical procedure may include one or more pre-surgical stages, one or more stages occurring during the surgical procedure, and/or one or more post-surgical stages. One or more of the stages may correspond to known surgical phases, such as preoperative, perioperative, intraoperative, postoperative, and post-discharge. Example pre-surgical stages may include wheeling a patient into an operating room, cleaning one or more items within the operating room, repositioning equipment within the operating room, setting up equipment within the operating room, preparing the operating room, preparing the surgical site, preparing the patient, placing a draping on the patient, intubating the patient, a surgical timeout, and/or preparing one or more mixtures. Some example post-surgical stages may include wheeling the patient out of the operating room, cleaning one or more items within the operating room, extubating the patient, dressing the patient, performing a surgical checklist, and/or documenting surgical notes. The images may be captured by one or more of cameras 146, camera 152, and/or video camera 140 and may depict one or more objects in medical environment 10. Step 902 may be performed by a subsystem that is the same or similar to image analysis subsystem 110 described above at least with respect to FIG. 1B.

At step 904, contextual information associated with the medical workflow may be determined based on the received images. For example, with reference to FIGS. 4A and 4B, images 302, image representations 454 of images 302 determined by medical stage ML model 320, and/or medical stage information 460, may be inputted to contextual ML model 310. Contextual ML model 310 may be configured to output contextual information 410. Contextual ML model 310 may be trained to detect and track objects within medical environment 10 (shown in FIG. 1A). For example, contextual ML model 310 may identify medical staff, patient 12, medical objects (e.g., sponges, medical tools, blood or other fluids), or other objects within medical environment 10. The contextual information may be determined based on the detected objects as well as any detected changes associated with those objects. For example, contextual information 410 of FIG. 4A may indicate when a quantity of surgical sponges used during the medical procedure has increased from a first value to a second value. As another example, contextual information 410 may indicate a volume of blood lost during the surgical procedure. The contextual information may instead or additionally be determined based on inputs from other devices within medical environment 10 (shown in FIG. 1A). For example, medical devices 120 within medical environment 10 may indicate cardiovascular data, respiratory data, neurological data, or other biometric information associated with patient 12, which may be used to determine and/or enhance contextual information 410. In another example, spoken inputs, gestures, and/or manual inputs may also be used to determine contextual information 410. For instance, a surgeon or other medical staff may dictate a notification that a surgical sponge has been used, which may be used to update contextual information 410. Step 904 may be performed by a subsystem that is the same or similar to image analysis subsystem 110.

At step 906, a stage of the medical workflow may be identified based on the images. One or more machine learning models may be used to identify the stage from the images. For example, medical stage ML model 320 of FIG. 4B may be provided with images 302, image representations 414 of FIG. 4A computed by contextual ML model 310, and/or contextual information 410 determined by contextual ML model 310. Medical stage ML model 320 may be trained to determine a current medical stage of the medical workflow based on images 302, image representations 454, and/or contextual information 410. The current medical stage may be determined based on contextual information 410, which may indicate when certain objects are being used and/or what surgical activities are being performed. For example, contextual information 410 indicating that a patient has entered medical environment 10 (shown in FIG. 1A) may indicate that medical workflow 200 of FIG. 2 is in first medical stage 202. The stage may instead or additionally be determined based on inputs detected from other devices. For example, verbal utterances indicating that a step of a checklist has been completed may be detected by microphones 16 (shown in FIG. 1A). Audio data representing the utterance may be used to determine the current medical stage of medical workflow 200. Step 906 may be performed by a subsystem that is the same or similar to image analysis subsystem 110.

At step 908, a subsequent stage of the medical workflow may be determined based on the identified stage. For example, in the instance the identified stage is first medical stage 202 (shown in FIG. 2), then the subsequent stage may be second medical stage 204. In addition to or instead of determining the subsequent stage, a subsequent task (or tasks) to be performed may be determined. For example, the identified stage may be used to determine a current activity or task being performed. Based on the current activity and/or task, a subsequent activity/task may be determined. Step 908 may be performed by a subsystem that is the same or similar to image analysis subsystem 110.

At step 910, first medical content associated with the contextual information may be retrieved. The first medical content may be stored in content database 168 (shown at least in FIG. 1B). The first medical content may be selected based on the determined contextual information. For example, first medical content 510 of FIGS. 5A-5B may be selected from content database 168 based on contextual information 512. For example, contextual information 512 may indicate a change in the sponge count, and first medical content 510 may include an updated number of surgical sponges used during the surgical procedure (e.g., displayed on a user interface). Step 910 may be performed by a subsystem that is the same or similar to content rendering subsystem 112 described above at least with respect to FIG. 1B.

At step 912, second medical content associated with the subsequent medical stage may be retrieved. The second medical content may be stored in content database 168 (shown at least in FIG. 1B). The second medical content may be selected based on the identified medical stage. For example, second medical content 520 of FIGS. 5A-5B may be selected from content database 168 based on medical stage information 522. Medical stage information 522 may indicate a current medical stage of a medical workflow, as well as a subsequent medical stage. The second medical content may be selected based on the subsequent medical stage. For example, as shown in FIG. 6, medical stage information 460 may indicate that the current stage is "Stage 1." Therefore, content associated with the subsequent stage information 602 (e.g., "Stage 2") may be retrieved (e.g., two checklists and a video). Step 912 may be performed by a subsystem that is the same or similar to content rendering subsystem 112.

At step 914, the first medical content and the second medical content may be presented. The first medical content and the second medical content may be presented via the same display device or using different display devices. For example, as illustrated in FIG. 8, medical content 804a may be presented via display device 802a, medical content 804b may be presented via display device 802b, and medical content 804c may be presented via display device 802c. A given display device may be selected for displaying medical content based on a location of the given display device within the medical environment, the type of content to be displayed, and/or one or more medical staff members located proximate those display devices. For example, medical content 804a may be presented via display device 802a based on one or more members of user group 806a being located proximate display device 802a. Step 914 may be performed by a subsystem that is the same or similar to content rendering subsystem 112.

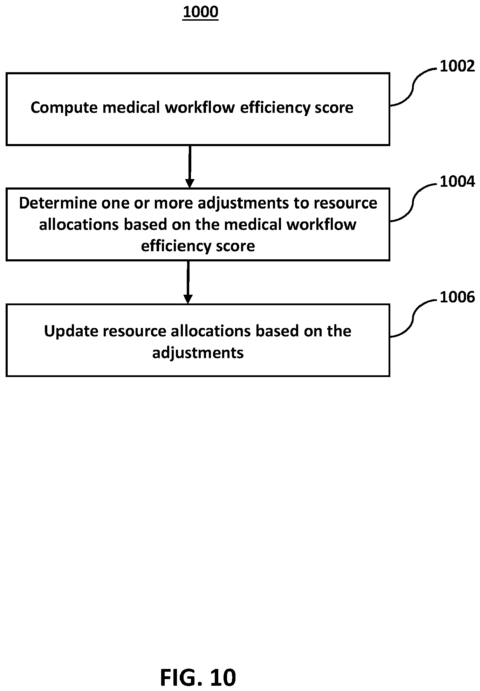

FIG. 10 illustrates a flowchart of an example process 1000 for improving a medical workflow efficiency score, according to some aspects. Process 1000 may begin at step 1002. At step 1002, a medical workflow efficiency score may be computed. The medical workflow efficiency score may be computed based on an amount of time needed to complete a medical procedure, an amount of time needed to complete one or more stages of a medical workflow, an amount of time that medical staff within a medical environment are detected to be idle during a medical workflow, an amount of time needed to complete one or more tasks performed during the stages of the medical workflow, or based on the amount of time needed to complete other activities associated with the medical workflow. These amounts of time may be compared with benchmark parameters of reference medical workflows, such as reference medical workflows stored in data structure 650 of FIG. 6. For example, the total amount of time needed to complete a current medical workflow may be compared to an average amount of time needed for other medical staff members to complete the same medical procedure. This comparison may be used to compute a medical workflow efficiency score. As another example, the reference medical workflows (e.g., RWF1, RWF2, and RWF3) may indicate an average amount of time required for other medical staff members to complete one or more stages of the medical workflow. The current amount of time needed for a medical staff member (e.g., a surgeon) to complete the same stage(s) may be compared to the average amount of time stored in reference medical workflows (e.g., RWF1, RWF2, RWF3, as seen in FIG. 6). This comparison may be used to compute an efficiency score for the medical workflow, a corresponding stage (or stages) of the medical workflow, and/or the medical staff member performing those one or more stages.

At step 1004, one or more adjustments to resource allocations may be determined based on the medical workflow efficiency score. For example, reference workflow information RWF1 of FIG. 6 may indicate that a predefined number of medical staff members (e.g., 1 or more, 2 or more, 5 or more, 10 or more, etc.) that should be allocated to assist with a given medical workflow. In the instance the medical workflow efficiency scores indicates that one or more medical staff members have remained idle for more than a threshold amount of time, it may be determined that the resource allocation of medical staff should be adjusted (e.g., the number of medical staff members may need to be decreased). As another example, the medical workflow efficiency scores of one or more medical staff members may indicate that the medical staff was active for more than a threshold amount of time, which may also indicate that the resource allocation of medical staff should be adjusted (e.g., the number of medical staff members may need to be increased). For example, the number of medical staff allocated for subsequent performances of the same or a similar medical procedure may be updated from the current number to an updated number. The updated number may be greater than or less than the current number.

At step 1006, the resource allocations may be updated based on the determined adjustments. For example, the number of medical staff allocated for the medical workflow may be adjusted from the current number (e.g., five medical staff) to an updated number (e.g., three medical staff).

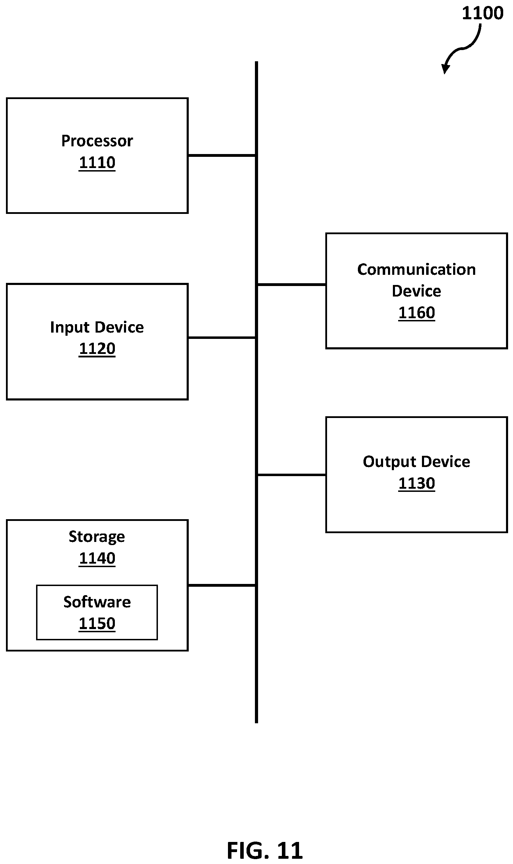

FIG. 11 illustrates an example computing system 1100, according to some aspects. Computing system 1100 may be used for performing any of the methods described herein, including processes 900 and 1000 of FIGS. 9 and 10, respectively, and can be used for any of the systems and/or devices described herein, including computing system 102 (and the subsystems included therein), medical device 120, client device 130, or other systems/devices described herein. Computing system 1100 can be a computer coupled to a network, which can be, for example, an operating room network or a hospital network. Computing system 1100 can be a client computer or a server. As shown in FIG. 11, computing system 1100 can be any suitable type of controller- (including a microcontroller) or processor- (including a microprocessor) based system, such as an embedded control system, personal computer, workstation, server, or handheld computing device (portable electronic device) such as a phone or tablet. The system can include, for example, one or more of processor 1110, input device 1120, output device 1130, storage 1140, or communication device 1160.

Input device 1120 can be any suitable device that provides input, such as a touch screen, keyboard or keypad, mouse, gesture recognition component of a virtual/augmented reality system, or voice recognition device. Output device 1130 can be or include any suitable device that provides output, such as a touch screen, haptics device, virtual/augmented reality display, or speaker.

Storage 1140 can be any suitable device that provides storage, such as an electrical, magnetic, or optical memory including a RAM, cache, hard drive, removable storage disk, or other non-transitory computer readable medium. Communication device 1160 can include any suitable device capable of transmitting and receiving signals over a network, such as a network interface chip or device. The components of the computer can be coupled in any suitable manner, such as via a physical bus or wirelessly.

Software 1150, which can be stored in storage 1140 and executed by processor 1110, can include, for example, the programming that embodies the functionality of the present disclosure (e.g., as embodied in the devices as described above). For example, software 1150 can include one or more programs for performing one or more of the steps of the methods disclosed herein.

Software 1150 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 1140, that can contain or store programming for use by or in connection with an instruction execution system, apparatus, or device.

Software 1150 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate or transport programming for use by or in connection with an instruction execution system, apparatus, or device. The transport readable medium can include, but is not limited to, an electronic, magnetic, optical, electromagnetic, or infrared wired or wireless propagation medium.

Computing system 1100 may be coupled to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

Computing system 1100 can implement any operating system suitable for operating on the network. Software 1150 can be written in any suitable programming language, such as C, C++, C#, Java, or Python. In various examples, application software embodying the functionality of the present disclosure can be deployed in different configurations, such as in a client/server arrangement or through a Web browser as a Web-based application or Web service, for example.

As used throughout this application, the word "may" is used in a permissive sense (i.e., meaning having the potential to), rather than the mandatory sense (i.e., meaning must). The words "include", "including", and "includes" and the like mean including, but not limited to. As used throughout this application, the singular forms "a," "an," and "the" include plural referents unless the content explicitly indicates otherwise. Thus, for example, reference to "an element" or "a element" includes a combination of two or more elements, notwithstanding use of other terms and phrases for one or more elements, such as "one or more." The term "or" is, unless indicated otherwise, non-exclusive, i.e., encompassing both "and" and "or." Terms describing conditional relationships, e.g., "in response to X, Y," "upon X, Y," "if X, Y," "when X, Y," and the like, encompass causal relationships in which the antecedent is a necessary causal condition, the antecedent is a sufficient causal condition, or the antecedent is a contributory causal condition of the consequent, e.g., "state X occurs upon condition Y obtaining" is generic to "X occurs solely upon Y" and "X occurs upon Y and Z." Such conditional relationships are not limited to consequences that instantly follow the antecedent obtaining, as some consequences may be delayed, and in conditional statements, antecedents are connected to their consequents, e.g., the antecedent is relevant to the likelihood of the consequent occurring. Statements in which a plurality of attributes or functions are mapped to a plurality of objects (e.g., one or more processors performing steps A, B, C, and D) encompasses both all such attributes or functions being mapped to all such objects and subsets of the attributes or functions being mapped to subsets of the attributes or functions (e.g., both all processors each performing steps A-D, and a case in which processor 1 performs step A, processor 2 performs step B and part of step C, and processor 3 performs part of step C and step D), unless otherwise indicated. Further, unless otherwise indicated, statements that one value or action is "based on" another condition or value encompass both instances in which the condition or value is the sole factor and instances in which the condition or value is one factor among a plurality of factors. Unless otherwise indicated, statements that "each" instance of some collection have some property should not be read to exclude cases where some otherwise identical or similar members of a larger collection do not have the property, i.e., each does not necessarily mean each and every. Limitations as to a sequence of recited steps should not be read into the claims unless explicitly specified, e.g., with explicit language like "after performing X, performing Y," in contrast to statements that might be improperly argued to imply sequence limitations, like "performing X on items, performing Y on the X'ed items," used for purposes of making claims more readable rather than specifying sequence. Statements referring to "at least Z of A, B, and C," and the like (e.g., "at least Z of A, B, or C"), refer to at least Z of the listed categories (A, B, and C) and do not require at least Z units in each category. Unless specifically stated otherwise, as apparent from the discussion, it is appreciated that throughout this specification discussions utilizing terms such as "processing," "computing," "calculating," "determining" or the like refer to actions or processes of a specific apparatus, such as a special purpose computer or a similar special purpose electronic processing/computing device. Features described with reference to geometric constructs, like "parallel," "perpendicular/orthogonal," "square", "cylindrical," and the like, should be construed as encompassing items that substantially embody the properties of the geometric construct, e.g., reference to "parallel" surfaces encompasses substantially parallel surfaces. The permitted range of deviation from Platonic ideals of these geometric constructs is to be determined with reference to ranges in the specification, and where such ranges are not stated, with reference to industry norms in the field of use, and where such ranges are not defined, with reference to industry norms in the field of manufacturing of the designated feature, and where such ranges are not defined, features substantially embodying a geometric construct should be construed to include those features within 15% of the defining attributes of that geometric construct. The terms "first", "second", "third," "given" and so on, if used in the claims, are used to distinguish or otherwise identify, and not to show a sequential or numerical limitation. As is the case in ordinary usage in the field, data structures and formats described with reference to uses salient to a human need not be presented in a human-intelligible format to constitute the described data structure or format, e.g., text need not be rendered or even encoded in Unicode or ASCII to constitute text; images, maps, and data-visualizations need not be displayed or decoded to constitute images, maps, and data-visualizations, respectively; speech, music, and other audio need not be emitted through a speaker or decoded to constitute speech, music, or other audio, respectively.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of the patents and publications referred to in this application are hereby incorporated herein by reference.

## Claims

1. A method for evaluating a medical workflow, comprising:
receiving one or more images depicting a medical environment and a medical workflow performed in the medical environment;
determining contextual information associated with the medical workflow based on the one or more images;
identifying a stage of the medical workflow based on the one or more images;
determining a subsequent stage of the medical workflow based on the identified stage; and
presenting, to one or more medical staff located within the medical environment, first medical content associated with the contextual information and second medical content associated with the subsequent stage.

2. The method of claim 1, further comprising:
generating a report for the medical workflow based on the contextual information.

3. The method of any one of claims 1-2, wherein the medical workflow comprises a first stage occurring prior to a beginning of a medical procedure, a second stage occurring during the medical procedure, and a third stage occurring after the medical procedure has been completed, wherein identifying the stage of the medical workflow comprises:
determining whether the one or more images were captured during the first stage, the second stage, or the third stage.

4. The method of any one of claims 1-3, further comprising:
inputting the one or more images into one or more machine learning models to obtain one or more image representations, wherein optionally a) identifying the stage of the medical workflow comprises:
identifying activities of the one or more medical staff based on the one or more image representations, the stage of the medical workflow being determined based on the identified activities and/or optionally b) determining the contextual information comprises:
detecting, based on the one or more image representations, one or more objects present within the one or more images; and
generating the contextual information based on the one or more detected objects.

5. The method of any one of claims 1-4, further comprising:
generating a report comprising at least some of the first medical content, the second medical content, or the first medical content and the second medical content; and
presenting, via one or more display devices, the report to the one or more medical staff.

6. The method of any one of claims 1-5, further comprising:
generating an audio message based on subsequent stage information, the audio message describing the subsequent stage of the medical workflow, and optionally further comprising:
outputting the audio message describing the subsequent stage to the one or more medical staff.

7. The method of any one of claims 1-6, wherein presenting the second medical content comprises:
selecting one or more display devices with which to present the second medical content, the one or more display devices being located within the medical environment; and
sending the second medical content to the one or more selected display devices;
wherein optionally selecting the one or more display devices comprises:
identifying, based on the one or more images, one or more relevant medical staff located within the medical environment, the one or more relevant medical staff associated with the subsequent stage; and
identifying, based on the one or more images, one or more displays located within the medical environment, the one or more displays proximate to the one or more relevant medical staff, wherein further optionally identifying the one or more relevant medical staff comprises:
detecting, using one or more machine learning models, activities of the one or more relevant medical staff based on the one or more images, the one or more relevant medical staff being identified from the one or more medical staff based on the detected activities.

8. The method of any one of claim 1-7, further comprising:
detecting, using one or more machine learning models, activities of the one or more medical staff during the stage of the medical workflow based on the one or more images; and
generating a report for the medical workflow based on the detected activities;
wherein optionally generating the report comprises:
generating an efficiency score indicating an efficiency of the medical workflow, the report comprising the generated efficiency score, wherein further optionally generating the efficiency score comprises:
determining a number of medical staff associated with the stage of the medical workflow based on the detected activities of the one or more medical staff; and
comparing the number of medical staff to a predefined number of medical staff to be used for the stage of the medical workflow, the efficiency score being based at least in part on a result of the comparison.

9. The method of any one of claims 1-8, further comprising:
detecting, using one or more machine learning models, activities of the one or more medical staff during the stage of the medical workflow based on the one or more images;
updating at least one of the first medical content or the second medical content based on the activities; and
presenting, using one or more display devices, at least one of the updated first medical content or the updated second medical content to the one or more medical staff.

10. The method of any one of claims 1-9, further comprising:
determining, based on the contextual information, that the medical workflow has progressed from a first stage to a second stage; and
updating the first medical content based on the medical workflow progressing from the first stage to the second stage.

11. The method of any one of claims 1-10, wherein presenting the first medical content and the second medical content comprises:
identifying at least one of (i) one or more checklists associated with the medical workflow or (ii) one or more videos associated with the medical workflow; and
generating at least one of the first medical content or the second medical content based on the at least one of (i) the one or more checklists or (ii) the one or more videos.

12. The method of any one of claims 1-11, further comprising:
identifying, based on the one or more images, a current task of a plurality of tasks associated with the identified stage;
determining, based on the one or more images, that the current task has been completed; and
modifying a checklist associated with the identified stage to indicate that the current task has been completed.

13. The method of any one of claims 1-12, further comprising:
identifying, from a plurality of tasks associated with the identified stage, a current task and a subsequent task;
determining, based on the one or more images, that the subsequent task has been started by the one or more medical staff prior to completion of the current task; and
sending a notification to the one or more medical staff indicating that the current task has not been completed, wherein optionally sending the notification to the one or more medical staff comprises:
generating and outputting at least one of an audible alert, a visual alert, or a haptic alert to the one or more medical staff.

14. The method of any one of claims 1-13, further comprising:
detecting, using one or more machine learning models, activities of the one or more medical staff during the stage of the medical workflow based on the one or more images; and
generating a report indicating that a quantity of the one or more medical staff is to be adjusted, wherein optionally the quantity of the one or more medical staff being adjusted comprises:
increasing the quantity of medical staff for the identified stage; or
decreasing the quantity of medical staff for the identified stage.

15. A system, comprising one or more processors programmed to perform a method comprising:
receiving one or more images depicting a medical environment and a medical workflow performed in the medical environment;
determining contextual information associated with the medical workflow based on the one or more images;
identifying a stage of the medical workflow based on the one or more images;
determining a subsequent stage of the medical workflow based on the identified stage;
presenting, to one or more medical staff located within the medical environment, first medical content associated with the contextual information and second medical content associated with the subsequent stage; and
optionally comprising the methods of any one of claims 2-14.
